(19) Europäisches Patentamt
European Patent Office
Office européen des brevets

(11) **EP 3 460 478 A1**

(12) **EUROPEAN PATENT APPLICATION**
published in accordance with Art. 153(4) EPC


(43) Date of publication:
**27.03.2019 Bulletin 2019/13**

(21) Application number: **17799449.8**

(22) Date of filing: **17.05.2017**

(51) Int Cl.:
***G01N 33/68*** *(2006.01)*

(86) International application number:
**PCT/JP2017/018592**

(87) International publication number:
**WO 2017/200025 (23.11.2017 Gazette 2017/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.05.2016 JP 2016099163**



(71) Applicants:
- **Osaka University**
**Suita-shi, Osaka 565-0871 (JP)**
- **Shiseido Company, Ltd.**
**Tokyo 104-0061 (JP)**

(72) Inventors:
- **ISAKA, Yoshitaka**
**Suita-shi**
**Osaka 565-0871 (JP)**
- **KIMURA, Tomonori**
**Suita-shi**
**Osaka 565-0871 (JP)**
- **YASUDA, Keiko**
**Suita-shi**
**Osaka 565-0871 (JP)**
- **HAMASE, Kenji**
**Fukuoka-shi**
**Fukuoka 812-8581 (JP)**
- **MITA, Masashi**
**Tokyo 104-0061 (JP)**

(74) Representative: **Scholz, Volker**
**Boehmert & Boehmert**
**Anwaltspartnerschaft mbB**
**Pettenkoferstrasse 22**
**80336 München (DE)**


(54) **BLOOD SAMPLE ANALYSIS METHOD AND SYSTEM, FOR DETERMINING DIABETES**


(57) The purpose of the present invention is to provide: a blood sample analysis method for determining diabetes complications in subjects with kidney damage on the basis of D- and L-amino acid content, from a blood sample; a method for examining diabetes complications; and a sample analysis system that outputs pathological information about diabetes complications. The purpose of this invention is achieved by using at least one amino acid selected from the group consisting of D-aspartic acid, D-proline, L-glutamine, and L-isoleucine, in order to determine diabetes complications.


FIG. 1-A

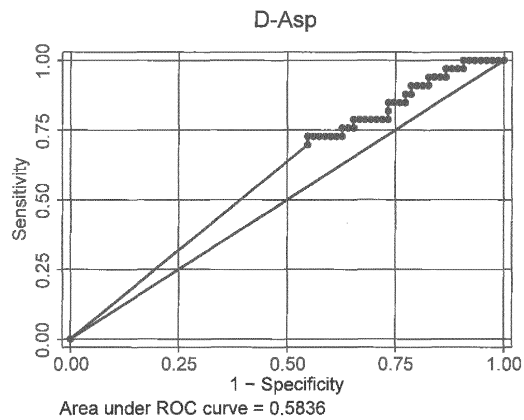


EP 3 460 478 A1

## Description

FIELD

The present invention relates to a blood sample analysis method for determining diabetes based on the amounts of D-form and L-form amino acids present in a blood sample, a method for testing for diabetes, and a sample analysis system that outputs pathological information relating to diabetes.

BACKGROUND

Diabetes is a metabolic disorder characterized by hyperglycemia that is generally classified into type 1 diabetes and type 2 diabetes. Type 1 diabetes is an illness that occurs as a result of selective destruction of β cells following the induction of an immune response caused by genetic factors or viral infection, and although it normally occurs due to autoimmunity, it is also known to rarely occur suddenly. Type 2 diabetes is a type of diabetes that has two causes consisting of reduced insulin secretion and decreased insulin sensitivity. Although type 2 diabetes is classified as lifestyle disease, the cause thereof is not fully understood. Type 2 diabetes is thought to occur as a result of persons having a constitution that is genetically susceptible to diabetes (genetic factors) leading a lifestyle that puts them at risk for diabetes (environmental factors).

Insulin primarily has the action of suppressing blood sugar levels, and has the action of suppressing blood sugar levels and promoting the neogenesis of glycogen, fats and various other types of reserve substances through promoting the uptake of glucose, amino acids and potassium and promoting protein synthesis in skeletal muscle, suppressing gluconeogenesis, promoting the synthesis and inhibiting the degradation of glycogen in the liver, and promoting the uptake and utilization of sugar, promoting the synthesis and inhibiting the degradation of fat in adipose tissue. In healthy individuals, blood sugar levels are always maintained within a fixed range through the action of insulin. Although blood sugar is important as an energy source, highly concentrated glucose causes a saccharification reaction by reacting with protein in the body due to the high reactivity of the aldehyde group thereof, thereby bringing about harmful actions in the body. Consequently, excessive blood sugar levels caused by abnormalities in insulin homeostasis cause neurological disorders and microvascular disease resulting in the onset of such disorders as diabetic neuropathy, diabetic retinopathy or diabetic nephropathy. Ultimately, diabetes can lead to numerous complications including blindness, skin ulceration, limb amputation, heart disease and kidney disease.

A protein biomarker in the form of hemoglobin A1C (HbA1C) is normally used to diagnosis diabetes. Since hemoglobin has a long lifetime in the blood, HbA1C fulfills the role of serving as a long-term indicator of blood sugar management. On the other hand, although HbA1C is used to confirm the efficacy of diabetes treatment in patients, contradictory results have occurred in patients undergoing treatment. Thus, normally HbA1C and fasting blood glucose level are respectively used in combination as markers to determine diabetes. While diabetes is diagnosed in the case both markers indicate diabetes, in cases in which only one of the markers indicates diabetes, a diagnosis of diabetes is made only after further observing typical symptoms of diabetes. Thus, diagnosis ultimately depends on the interpretation and judgment of the diagnosing physician. In addition, since Hb1AC is not suited for assessment of short-term therapeutic effects in patients having undergone diabetes treatment, therapeutic efficacy is assessed and progress is monitored by combining the use of a short-term diabetes marker such as 1,5-anhydro-D-glucitol or glucoalbumin.

Thus, there is a desire for the development of a diabetes marker that enables diagnosis while eliminating the interpretation and judgment of a physician, and although novel diabetes markers present in biological samples, such as ApoCIII protein, aminoacyl-tRNA synthetase or OLMF4 polypeptide, have been found (PTL 1 to 3), satisfactory markers able to be used in place of Hb1AC have yet to be obtained.

Diabetic nephropathy is known to be a diabetic complication. When blood sugar levels remain persistently high due to diabetes, glomeruli of the kidneys are damaged resulting in decreased renal function. Diabetes is an example of one of the causes of chronic kidney disease, and since the treatment strategy differs between kidney disease patients complicated with diabetes and kidney disease patients not complicated with diabetes, it is necessary that diabetic complication be diagnosed both simply and highly accurately in kidney disease patients. Although urinary albumin value or urinary albumin/creatinine ratio is used as a marker for complication with diabetes in kidney disease patients, these markers have problems in terms of quantitative performance, sensitivity and cost.

D-amino acids, which have conventionally been thought to not exist in the bodies of mammals, have been determined to be present in various tissues accompanying advances made in the field of detection technology (PTL 4), and these D-amino acids have been predicted to be responsible for some form of physiological function. Several D-amino acids present in body fluids have been determined to fluctuate independently of L-amino acids, and have been shown to fluctuate corresponding to the type of disease (PTL 5). Although PTL 5 investigates fluctuations in D-amino acids and L-amino acids in diabetes patients, among these 40 types of chiral amino acids, D-alanine, L-cysteine and L-glutamic acid were confirmed to fluctuate in diabetes patients, while fluctuations in other amino acids were unable to be confirmed.

[CITATION LIST]

[PATENT LITERATURE]

[PTL1] Japanese Patent No. 5876826
[PTL2] Japanese Patent No. 5571696
[PTL3] Japanese Patent No. 5698254
[PTL4] Japanese Patent No. 4291628
[PTL5] International Publication No. WO 2013/140785

[NON PATENT LITERATURE]

[NPLl]Transl. Res. 2012 Apr; 159(4): 303-12
[NPL2] International Diabetes Federation: Managing Older People with Type 2 Diabetes, Global Guidelines

SUMMARY

[TECHNICAL PROBLEM]

There is a desire for the development of a technology for identifying and analyzing diagnostic markers able to take the place of existing diagnostic markers for diabetes such as fasting blood glucose level or Hb1AC as well as markers for diagnosing diabetic complication in kidney disease such as urinary protein albumin or urinary albumin/creatinine ratio, as well as technology for accurately determining, testing or diagnosing diabetes through the use thereof.

[SOLUTION TO PROBLEM]

The inventors of the present invention found that, when chiral amino acids were analyzed in the blood of cohorts suffering from kidney disease, several chiral amino acids surprisingly fluctuated in association with diabetes in the cohorts, thereby leading to completion of the present invention.
Thus, the present invention relates to a blood sample analysis method for determining diabetes, wherein diabetes can be determined based on the amount of at least one type of amino acid among chiral amino acids.
In another aspect thereof, the present invention relates to a blood sample analysis system capable of carrying out the analysis method of the present invention. This type of sample analysis system contains a storage unit, an input unit, an analysis/measurement unit, a data processing unit and an output unit, and analyzes blood samples followed by outputting pathological information relating to diabetes.
In still another aspect, the present invention relates to a program able to be installed in the sample analysis system of the present invention and to a storage medium that stores that program.

[ADVANTAGEOUS EFFECTS OF INVENTION]

The present invention is able to provide a novel diabetes marker capable of fluctuating according to a principle that differs from that of albumin, which appears in the urine due to protein saccharification or decreased renal function, and enables highly accurate determination of diabetes by using a plurality of chiral amino acids in combination.

BRIEF DESCRIPTION OF DRAWINGS

FIG. 1-A indicates ROC curves for the sensitivity and specificity of diagnosing diabetes by D-Asp.
FIG. 1-B indicates ROC curves for the sensitivity and specificity of diagnosing diabetes by D-Pro.
FIG. 1-C indicates ROC curves for the sensitivity and specificity of diagnosing diabetes by L-Gln.
FIG. 1-D indicates ROC curves for the sensitivity and specificity of diagnosing diabetes by L-Ile.

FIG. 2-A indicates the amounts of D-Asp in subjects suffering from diabetes and subjects not suffering from diabetes.
FIG. 2-B indicates the amounts of D-Pro in subjects suffering from diabetes and subjects not suffering from diabetes.
FIG. 2-C indicates the amounts of L-Gln in subjects suffering from diabetes and subjects not suffering from diabetes.
FIG. 2-D indicates the amounts of L-Ile in subjects suffering from diabetes and subjects not suffering from diabetes.
FIG. 3-A indicates (1) a graph indicating the correlation between age and D-Ala level, (2) a graph indicating ROC curves between age and D-Ala level, and (3) a graph indicating ROC curves between age and D/L% value.
FIG. 3-B indicates (1) a graph indicating the correlation between age and D-Pro level, (2) a graph indicating ROC curves between age and D-Pro level, and (3) a graph indicating ROC curves between age and D/L% value.
FIG. 3-C indicates (1) a graph indicating the correlation between age and D-alloIle level and (2) a graph indicating ROC curves between age and D-alloIle level.
FIG. 3-D indicates (1) a graph indicating the correlation between age and D-Leu level and (2) a graph indicating ROC curves between age and D-Leu level.
FIG. 3-E indicates (1) a graph indicating the correlation between age and L-Ile level and (2) a graph indicating ROC curves between age and L-Ile level.
FIG. 3-F indicates (1) a graph indicating the correlation between age and L-Ser level and (2) a graph indicating ROC curves between age and L-Ser level.
FIG. 4-A is a graph indicating the correlation between estimated glomerular filtration rate (eGFR) and (1) L-Asn level, (2) D-Asn level and (3) D/L-Asn value.
FIG. 4-B is a graph indicating the correlation between estimated glomerular filtration rate (eGFR) and (1) L-Ser level, (2) D-Ser level and (3) D/L-Ser value.
FIG. 4-C is a graph indicating the correlation between estimated glomerular filtration rate (eGFR) and (1) L-Asp level, (2) D-Asp level and (3) D/L-Asp value.
FIG. 4-D is a graph indicating the correlation between estimated glomerular filtration rate (eGFR) and (1) L-Ala level, (2) D-Ala level and (3) D/L-Ala value.
FIG. 4-E is a graph indicating the correlation between estimated glomerular filtration rate (eGFR) and (1) L-Ile level and (2) D-alloIle level.
FIG. 4-F is a graph indicating the correlation between estimated glomerular filtration rate (eGFR) and (1) L-Phe level, (2) D-Phe level and (3) D/L-Phe value.
FIG. 4-G is a graph indicating the correlation between estimated glomerular filtration rate (eGFR) and (1) L-Lys level, (2) D-Lys level and (3) D/L-Lys value.
FIG. 4-H is a graph indicating the correlation between estimated glomerular filtration rate (eGFR) and (1) L-Thr level and (2) D-alloThr level.
FIG. 4-I is a graph indicating the correlation between estimated glomerular filtration rate (eGFR) and (1) L-Pro level, (2) D-Pro level and (3) D/L-Pro value.
FIG. 4-J is a graph indicating the correlation between estimated glomerular filtration rate (eGFR) and (1) L-Leu level, (2) D-Leu level and (3) D/L-Leu value.
FIG. 4-K is a graph indicating the correlation between estimated glomerular filtration rate (eGFR) and (1) L-Trp level.
FIG. 4-L is a graph indicating the correlation between estimated glomerular filtration rate (eGFR) and (1) L-Tyr level.
FIG. 4-M is a graph indicating the correlation between estimated glomerular filtration rate (eGFR) and (1) L-His level, (2) D-His level and (3) D/L-His value.
FIG. 5 is a schematic diagram of the analysis system of the present invention.
FIG. 6 is a flow chart indicating an example of operation for determining diabetic complication.
FIG. 7 is a flow chart indicating an example of operation for determining kidney disease and diabetic complication.

## DESCRIPTION OF EMBODIMENTS

The present invention relates to a blood sample analysis method for determining diabetes that comprises a step for measuring the amount of at least one type of chiral amino acid, and a step for determining diabetes based on the measured amount of the at least one type of amino acid. According to the analysis method of the present invention, the present invention can also be said to be a diagnostic method in another aspect since it enables assessment of the pathology of diabetes.

The step for measuring the amount of chiral amino acid is directed to only measuring the amount of a target amino acid or collectively measuring other chiral amino acids as well. In addition, chiral amino acids can also serve as diagnostic markers for other diseases. Thus, the D-forms and L-forms of twenty types of proteinogenic amino acids present in a blood sample are preferably measured collectively from the viewpoint of analyzing a plurality of diseases all at once. A step for acquiring the blood sample and a step for treating the acquired blood sample may also be carried out prior to the step for measuring the amount of chiral amino acid. The blood sample may be any arbitrary sample provided it is a sample derived from blood, such as whole blood, serum or plasma.

The step for determining diabetes makes it possible to determine the presence or absence of affliction with diabetes by comparing the amount of a specific chiral amino acid with a cutoff value. Whether a cutoff value is exceeded on the high side or low side can be suitably selected corresponding whether the chiral amino acid used increases or decreases in the case of suffering from diabetes. For example, since levels decrease in diabetes patients in the case of D-Asp, D-Pro and L-Gln, a patient can be determined to be suffering from diabetes in the case of belonging to the low group, while a patient can be determined to not be suffering from diabetes in the case of belonging to the high group. On the other hand, since the level of L-Ile increases in diabetes patients, a patient can be determined to be suffering from diabetes in the case of belonging to the high group, and can be determined to not be suffering from diabetes in the case of belonging to the low group. Determination may be made based only on the measured amount of a chiral amino acid or may be made using an index value obtained by processing the measured amount of a chiral amino acid with an arbitrary variable or constant. Thus, measured values include index values determined from those measured values. In the present invention, an index value may be the measured amount of an amino acid, may be calculated based on a measured amount, for example, may be a concentration ratio or proportion and the like with a corresponding isomer (such as the L-form in the case of the D-form or the D-form in the case of the L-form). Any arbitrary variable capable of having an effect on the amount of chiral amino acid can be used as a variable, such as age, body weight, gender or BMI, in addition to the amount of the corresponding isomer.

Determination of diabetes consists of classifying the amount of at least one chiral amino acid present in a blood sample into two or more groups based on a cutoff value followed by determining the presence of diabetes according to that classification. Since the inventors of the present invention has found that diabetes occurs when the amount of a specific chiral amino acid in the blood demonstrated a high value or low value, a subject can be determined to be suffering from diabetes in the case that subject belongs to a high value group or low value group. Thus, determination can be carried out by a medical assistant who is not a physician or can be carried out by an analysis laboratory and the like. Thus, the analysis method of the present invention can be said to be a preliminary or auxiliary method for making a diagnosis.

In another aspect of the present invention, the analysis method of the present invention may include a step for calculating a pathological index value for determining diabetes instead of the step for determining diabetes. Diabetes can be determined to be occurring by comparing the pathological index value calculated as a result of this analysis method with a predetermined cutoff value.

In the present invention, a specific chiral amino acid used to determine diabetes refers to the D-form or L-form of a proteinogenic amino acid. Since the D-form and L-form have different internal kinetics and metabolism, prognosis can be predicted with high accuracy by distinguishing between the D-form and L-form. Examples of proteinogenic amino acids include alanine (Ala), arginine (Arg), asparagine (Asn), aspartic acid (Asp), cysteine (Cys), glutamine (Gin), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val). Among these, at least one type of amino acid selected from the group consisting of D-aspartic acid (D-Asp), D-proline (D-Pro), L-glutamine (L-Gln) and L-isoleucine (L-Ile) are preferable from the viewpoint of determining diabetes with higher accuracy. At least one type of amino acid selected from the group consisting of D-aspartic acid (D-Asp), D-proline (D-Pro), L-glutamine (L-Gln) and L-isoleucine (L-Ile), or an arbitrary combination thereof, are used more preferably.

The borderline for determining diabetes can also be arbitrarily determined by analyzing cohorts and performing statistical processing. A method commonly known among persons with ordinary skill in the art may be used for the statistical processing method, examples of which include ROC analysis and the t-test, or the mean, median and X percentile values of a healthy subject group or diabetes patient group can also be used. Here, an arbitrary value can be selected for X, and a value of 3, 5, 10, 15, 20, 30, 40, 60, 70, 80, 85, 90, 95 or 97 can be suitably used. The cutoff value may consist of a single cutoff value or pathology may be classified according to the severity of the disease. The cutoff value used to determine a borderline differs according to the type of cohort, and as an example thereof, the cutoff value that can be used to determine diabetes by ROC analysis of cohorts used in examples of the present application is 0.1 μg/ml in the case of D-aspartic acid, 2.5 μg/ml in the case of D-proline, 665 μg/ml in the case of L-glutamine and 49.3 μg/ml in the case of L-isoleucine. The onset of diabetes can be determined in the case blood D-amino acid concentration of a subject is higher or lower than these cutoff values. However, the cutoff values used are not intended to be limited to the aforementioned cutoff values.

A subject refers to an arbitrary subject, such as a healthy individual or person having the possibility of being afflicted with diabetes, on whom the analysis method of the present invention can be carried out. In a certain aspect, since the analysis method of the present invention is used in medical examinations, the subject may include arbitrary subjects.

In another aspect of the present invention, a subject is a subject suffering from kidney disorder. In this case, the determination of diabetes according to the present invention makes it possible to determine whether or not a subject who has been determined as suffering from kidney disorder suffers from diabetes as complication. Thus, in an aspect, a subject in the present invention refers to a patient who has been diagnosed or determined as having kidney disorder.

Renal disorder refers to a condition .having decreased renal function, and is mainly divided into acute kidney disease and chronic kidney disease. Although decreased renal function is determined by decreased glomerular filtration rate (GFR), it may be determined by estimated glomerular filtration rate (eGFR) calculated based on variable, such as age, sex, etc., from creatinine value. In a further aspect, it may be determined by a kidney disorder marker demonstrating decreased renal function, such as serum creatinine concentration, KIM-1, NGAL, etc. In a further aspect, the amount of at least one amino acid used for determination of kidney disorder is measured in a patient who have been determined as having kidney disorder, the kidney disorder is determined in advance or at the same time based on the measured amount.

Thus, in another aspect of the present invention, the present invention relates to a blood sample analysis method for the purpose of determining kidney disease and determining the presence of a diabetic complication in any subject. This analysis method includes a step for measuring the amount of at least one chiral amino acid used to determine kidney disease and measuring the amount of at least one chiral amino acid used to determine diabetic complication, a step for determining kidney disease based on the measured amount of the at least one chiral amino acid used to determine kidney disease, and a step for determining the presence of a diabetic complication based on the measured amount of the at least one chiral amino acid used to determine diabetic complication in the case where a subject is determined to have kidney di sease..

In order to determine diabetes, it was conventionally required to either use an existing diagnostic marker of diabetes such as fasting blood glucose level or Hb1AC or measure urinary albumin level or urinary albumin/creatinine ratio in a patient diagnosed with kidney disease. Thus, although it was necessary to respectively analyze kidney disease markers and diabetes markers in order to diagnose a diabetic complication in a patient suffering from kidney disease, use of the present invention makes it possible to determine the presence of a diabetic complication after having determined kidney disease based on previously acquired chiral amino acid levels.

Determination of kidney disease is carried out based on the amount of at least one chiral amino acid present in a blood sample.

According to the inventors of the present invention, since the amount of at least one chiral amino acid present in the blood selected from the group consisting of D-asparagine, D-serine, D-aspartic acid, D-allo-threonine, D-alanine, D-proline, D-leucine, L-histidine, L-serine, L-aspartic acid, L-alanine, L-isoleucine, L-phenylalanine, L-tryptophan, L-lysine and L-tyrosine were found to correlate with eGFR values (Figs. 4A to 4L), these chiral amino acids can be used to determine kidney disease. More specifically, kidney disease can be determined by assigning the amount of at least one chiral amino acid in a blood sample to two or more groups classified in advance, and in another aspect thereof, the severity of kidney disease can be determined.

The borderline for determining kidney disease can be arbitrarily determined by similarly analyzing cohorts and carrying out statistical processing. A method commonly known among persons with ordinary skill in the art may be used for the statistical processing method, examples of which include ROC analysis and the t-test, or the mean, median and X percentile values of a healthy subject group or patient group can be used. Here, an arbitrary value can be selected for X, and a value of 3, 5, 10, 15, 20, 30, 40, 60, 70, 80, 85, 90, 95 or 97 can be suitably used. The cutoff value may consist of a single cutoff value or pathology can be classified according to the severity of the disease. Examples of chiral amino acids used to determine kidney disease include D-asparagine, D-serine, D-aspartic acid, D-allo-threonine, D-alanine, D-proline, D-leucine, L-histidine, L-serine, L-aspartic acid, L-alanine, L-isoleucine, L-phenylalanine, L-tryptophan, L-lysine and L-tyrosine, and their respective cutoff values can be arbitrarily determined by cohort analysis. A subject can be determined to have kidney disease in the case the D-amino acid concentration in the blood of the subject is higher than the cutoff value.

In still another aspect of the present invention, the present invention relates to a method for determining an eGFR value based on the amount of at least one chiral amino acid in a blood sample. This method includes a step for measuring the amount of at least one chiral amino acid in a blood sample and a step for determining an eGFR value based on the measured amount of the at least one chiral amino acid. In one aspect thereof, the step for determining an eGFR value based on the measured value of a chiral amino acid can be used to determine the eGFR value based on a predetermined regression curve. In another aspect thereof, at the step for determining an eGFR value based on the measured value of a chiral amino acid, cohorts may be preliminarily divided into a plurality of groups corresponding to the amount of chiral amino acid followed by pre-correlating those groups with eGFR values or the range thereof and then classifying the measured values for the groups. Examples of such chiral amino acids that can be used include at least one amino acid selected from the group consisting of D-asparagine, D-serine, D-aspartic acid, D-allo-threonine, D-alanine, D-proline, D-leucine, L-histidine, L-serine, L-aspartic acid, L-alanine, L-isoleucine, L-phenylalanine, L-tryptophan, L-lysine and L-tyrosine.

Since chiral amino acids can also be used as kidney disease markers or diabetes markers according to the type thereof, comprehensively measuring chiral amino acids makes it possible to determine the presence of a diabetic complication after having determined kidney disease.

A chiral amino acid for which an increase or decrease is not observed in kidney disease patients is preferable in terms of determining the presence of diabetic complications in kidney disease patients, and at least one amino acid selected from the group consisting of D-aspartic acid (D-Asp), L-glutamine (L-Gln) and L-isoleucine (L-Ile), or an arbitrary combination thereof, is used.

A method commonly known among persons with ordinary skill in the art may be used to measure the amount of chiral amino acid in a sample. For example, the D-form and L-form of an amino acid can be measured simultaneously by a method consisting of preliminarily specifically derivatizing D- and L-amino acids in a stereoisomeric manner with o-phthalaldehyde (OPA), N-tert-butyloxycarbonyl-L-cysteine (Boc-L-Cys) or other modifying reagent followed by separating by means of an analysis column, such as ODS-80TsQA column, with using a mixture of 100 mM acetate buffer (pH 6.0) and acetonitrile for gradient elution. In addition, a method consisting of preliminarily derivatizing D- and L-amino acids with a fluorescent reagent, such as 4-fluoro-7-nitro-2,1,3-benzoxazole (NBD-F) followed by specifically separating each amino acid in non-stereoisomeric manner by means of an analysis column, such as ODS-80TsQA, Mightysil RP-18GP column, etc., and then optically resolving using a Pirkle-type chiral stationary phase column (such as the Sumichiral OA-2500S or OA-2500R), can be used to measure trace amounts of proteinogenic amino acids (Hamase, K. and Zaitsu, K., Analytical Chemistry, Vol. 53, 677-690 (2004)). An optical resolution column system in the present description refers to a separation and analysis system that at least uses an optical resolution column, and may include separation and analysis by an analysis column other than an optical resolution column. More specifically, the concentrations of D- and L-amino acids in a sample can be measured by using a method for analyzing optical isomers characterized in comprising a step for passing a sample containing a component having optical isomers through a first column filler serving as a stationary phase together with a fist liquid as a mobile phase to separate the components in the sample, a step for individually retaining each of the components of the sample in a multi-loop unit, a step for supplying each of the components of the sample retained in the multi-loop unit to a second column filler having an optically active center serving as a stationary phase together with a second liquid serving as a mobile phase to separate the optical isomers contained in each of the components of the sample through a flow path, and a step for detecting the optical isomers contained in each of the components of the sample (Japanese Patent No. 4291628). Alternatively, D-amino acids can be assayed by an immunological method using monoclonal antibody capable of identifying optical isomers of amino acids, such as monoclonal antibody that specifically binds with D-leucine or D-aspartic acid and the like (Japanese Unexamined Patent Publication No. 2009-184981).

In the present invention, the amount of chiral amino acid in a blood sample may be used alone to determine diabetes or may be used in combination with the amounts of one or more other chiral amino acids able to be used to determine diabetes. In addition, the analysis method of the present invention may further include a step for measuring a variable associated with diabetes, and can be used to determine diabetes by combining this variable with an amount of a chiral amino acid. Examples of such variables include history of diabetes, age, gender and fasting blood glucose level, while additional examples include known diabetes markers and diabetic complication markers. Examples of such known markers include Hb1AC, fasting blood glucose level, 1,5-anhydro-D-glucitol, glucoalbumin, urinary albumin and urinary albumin/creatinine ratio.

In the case of having determined diabetes or a complication of diabetes and kidney disease using the analysis method of the present invention, treatment is selected and performed that is suitable for diabetes and/or diabetic nephropathy. Although not limited thereto, it is necessary to further implement blood sugar management, lifestyle improvements and the like. As blood sugar management, medicinal treatment such as administration of biguanides, thiazolidine drugs, sulfonylurea drugs, insulin secretion promoters, DPP4 inhibitors, $\alpha$-glucosidase inhibitors or SGLT2 inhibitors, are carried out for the purpose of improvement of insulin resistance, promotion of insulin secretion, regulation of sugar absorption and excretion. Lifestyle improvements include, for example, quitting smoking, receiving guidance on exercise and dietary restrictions to lower BMI,. These treatment strategies are determined based on chiral amino acid levels after having undergone an interview with a physician. Thus, in another aspect, the present invention relates to a method for treating diabetic kidney disease comprising carrying out the analysis method of the present invention followed by further carrying out treatment of kidney disease occurring as a complication of diabetes. Details of the treatment method can be suitably selected with reference to, for example, NPL1 and NPL2. These documents are incorporated in the description.

In still another aspect thereof, the present invention relates to a method for determining the estimated age of a subject using chiral amino acids. This method includes a step for measuring the amount of at least one type of amino acid present in a blood sample selected from the group consisting of D-alanine, D-leucine, D-allo-isoleucine, D-proline, L-serine and L-isoleucine, and a step for determining age based on the measured amount of the at least one type of amino acid and a predetermined correlation curve between age and D-amino acid levels. This method is based on the finding that several chiral amino acids present in the blood demonstrate a correlation with age (Figs. 3A to 3F). Estimated age can be determined based on chiral amino acid levels for determining age by preliminarily acquiring regression curves or cutoff values between age and chiral amino acids for determining age among arbitrary cohorts, and then comparing with measured values. According to this method, age can be estimated in persons who have lost consciousness or dementia patients.

With respect to the cutoff values, in the case of calculating using the cohorts of the present invention, the cutoff value enabling identification of subjects over 70 years old, for example, can be selected from 4.7 μg/ml or more in the case of D-alanine (D-Ala), 2.5 μg/ml or more in the case of D-proline (D-Pro), 0.1 μg/ml or more in the case of D-allo-isoleucine (D-allo-Ile), 0.50 or more in the case of D-leucine (D-Leu), 134.6 or less in the case of L-serine (L-Ser) and 58.6 or less in the case of L-isoleucine (L-Ile). Cutoff values can be suitably selected with respect to age. Cutoff values can be suitably selected with respect to age.

The sample analysis system and program of the present invention are composed so as to carry out the method of the present invention. Fig. 5 is a block diagram of the sample analysis system of the present invention. This sample analysis system 10 contains a storage unit 11, an input unit 12, an analysis/measurement unit 13, a data processing unit 14 and an output unit 15, and is able to analyze a blood sample of a subject and output pathology information. More specifically, in the sample analysis system 10 of the present invention,
the storage unit 11 stores cutoff values of blood chiral amino acid levels for determining diabetes along with diabetes pathology information, which are input from the input unit 12,
the analysis/measurement unit 13 separates at least one chiral amino acid for determining diabetes among the proteinogenic amino acids present in the blood sample of the subject and assays the amount thereof,
the data processing unit 14 compares the measured amount of at least one chiral amino acid with the cutoff values stored in the storage unit 11 to determine diabetes information of the subject, and
the output unit 15 is able to output pathology information on the diabetes of the subject.

In the sample analysis system and program of the present invention,
the storage unit 11 stores cutoff values of blood chiral amino acid levels for determining kidney disease and kidney disease information which are input from the input unit 12,
the analysis/measurement unit 13 separates at least one chiral amino acid for determining kidney disease among the proteinogenic amino acids present in the blood sample of the subject and assays the amount thereof, and
the data processing unit 14 compares the measured amount of at least one chiral amino acid for determining kidney disease with the cutoff values of chiral amino acids for determining kidney disease stored in the storage unit 11 to determine kidney disease information of the subject, and
as a result thereof, the output unit 15 is able to output pathology information on diabetes together with information on kidney disease of the subject.

The storage unit 11 has a memory device such as RAM, ROM or flash memory, a stationary disk device such as a hard disk drive, or a portable storage device such as a flexible disk or optic disk. The storage unit stores data measured with the analysis/measurement unit, data and instructions input from the input unit12, the results of arithmetic processing performed with the data processing unit14, as well as a computer program used for various types of processing by an information processing device, and database and the like. The computer program may be installed via a computer-readable storage medium such as a CD-ROM or DVD-ROM or by accessing from the Internet. The computer program is installed in the storage unit using a known setup program and the like.

The input unit 12 functions as an interface and the like and contains an operating unit such as a keyboard or mouse. The input unit is able to input data measured with the analysis/measurement unit 13 and instructions and the like for arithmetic processing performed with the data processing unit 14. When the analysis/measurement unit 13 is present outside, in addition to the operating unit, the input unit 12 may also contain an interface unit that enables input of measured data and the like via a network or storage medium.

The analysis/measurement unit 13 carries out the step for measuring the amount of a chiral amino acid in a blood sample. Thus, the analysis/measurement unit 13 has a configuration that enables separation and measurement of chiral amino acids. Although one amino acid may be analyzed at a time, some or all types of amino acids can be analyzed collectively. Without intending to limit to that indicated below, the analysis/measurement unit 13 may be a high-performance liquid chromatography (HPLC) system comprising, for example, a sample introduction unit, optical resolution column and detection unit. The analysis/measurement unit 13 may be composed separate from the sample analysis system 10 or measured data and the like may be input via the input unit 12 using a network or storage medium.

The data processing unit 14 is composed so as to determine diabetic complication by comparing the measured amount of chiral amino acid with a cutoff value stored in the storage unit 11. The data processing unit 14 performs various types of arithmetic processing on data measured with the analysis/measurement unit 13 and stored in the storage unit 11 in accordance with a program stored in the storage unit 11. Arithmetic processing is carried out by a CPU contained in the data processing unit 14. This CPU contains a functional module that controls the analysis/measurement unit 13, input unit 12, storage unit 11 and output unit 15, and is able to carry out various types of control. Each of these units may be respectively and independently controlled with integrated circuits, microprocessors or firmware and the like.

The output unit 15 is composed so as to output pathology index values and/or pathology information, which are resulted from arithmetic processing with the data processing unit. The results of arithmetic processing in the data processing unit 14 may be output directly in the output unit 15, or may be stored in the storage unit 11 temporarily, and then be output in the output unit 15 as required. The output unit 15 may be an output means such as a printer or display device such as a liquid crystal display that directly displays the results of arithmetic processing, or may be an interface unit for output to an external storage device or output via a network.

Fig. 6 is a flow chart indicating an example of an operation for determining diabetes according to the program of the present invention.

Specifically, the program of the present invention is a program let an information processing device comprising containing an input unit 12, an output unit 15, a data processing unit 14 and a storage unit 11 to determine diabetic information. The program according to the present invention contains following commands to be executed by the aforementioned information processing device:

for the storage unit 11 storing cutoff values of blood chiral amino acid levels for determining kidney disease and kidney disease information which are input from the input unit 12,
for the storage unit 11 storing measured mount of at least one chiral amiono acid which is input from input unit 12,
for the data processing unit 14 comparing the measured amount stored in the storage unit 11 with the cutoff values stored in the storage unit, to determine diabetes information, and
for the output unit 15 outputting diabetes information.

The program of the present invention may be housed in a storage medium or may be provided via a telecommunication line such as the Internet or LAN.

Fig. 7 is a flow chart indicating an example of operation for determining kidney disease pathology information and the presence of diabetic complication by the program of the present invention.

Specifically, the program of the present invention is a program let an information processing device comprising containing an input unit 12, an output unit 1 5, a data processing unit 14 and a storage unit 11 to determine diabetic complication along with kidney disease pathogenic information The program of the present invention contains commands to be executed by the aforementioned information processing device:

for respectively storing predetermined cutoff values for at least one chiral amino acid for determining kidney disease and at least one chiral amino acid for determining diabetes which are input from the input unit 12 together with kidney disease and diabetic complication information,
for the storage unit 11 storing the measured amounts of at least one chiral amino acid for determining kidney disease and at least one chiral amino acid for determining diabetes which are input from the input unit 12,
for the data processing unit 14 comparing the cutoff values stored in the storage unit 11 with the measured values stored in the storage unit 11 to determine kidney disease and diabetic complication information, and
for the output unit 15 outputting the kidney disease and diabetes information. The program of the present invention may be housed in a storage medium or may be provided via a telecommunication line such as the Internet or LAN.

In another aspect of the present invention, the system of the present invention may be a system for determining estimated age. This system contains a storage unit, input unit, data processing unit and output unit, and is able to carry out the following step:

inputting the amount of at least one type of chiral amino acid present in the blood for determining age and a regression curve or cutoff value between the amount of the chiral amino acid and age from the input unit and storing in the storage unit,
inputting the measured value of at least one type of chiral amino acid in the blood sample of a subject from the input unit and storing in the storage unit,
determining the estimated age of the subject based on the stored measured value of the amount of the amino acid and the regression curve or cutoff value based on the data processing unit, and
outputting the estimated age to the output unit.

In another aspect of the present invention, the present invention relates to a system for determining eGFR values that carries out the method for determining eGFR values of the present invention. This system contains a storage unit, an input unit, a data processing unit and an output unit, and and is able to carry out the following step:

inputting the amounts of at least type of one amino acid among chiral amino acids present in the blood for determining eGFR values and a regression curve or cutoff values of those eGFR values from the input unit and storing in the storage unit,
inputting the measured values of the amount of at least one type of amino acid among chiral amino acids present in a blood sample of a subject from the input unit and storing in the storage unit,
determining the eGFR value of the subject based on the stored measured value of the amount of the aforementioned amino acid and the aforementioned regression curve or cutoff value by the data processing unit, and

outputting that eGFR value to the output unit.

In the case the data processing device is provided with an analysis/measurement unit 13, instead of inputting values of the amounts of at least one chiral amino acid from the input unit 12, the analysis/measurement unit 13 may contain commands for having the information processing device to execute separation and measurement of chiral amino acids from a blood sample and storage of the measured values in the storage unit 11.

All documents mentioned in the present description are incorporated in their entirety herein by reference.

Examples of the present invention as explained below are indicated for the purpose of exemplification only, and do not limit the technical scope of the present invention. The technical scope of the present invention is only limited by the scope of claim for patent. The present invention can be modified, such as by adding, deleting or substituting constituents of the present invention, on the condition that such modification does not deviate from the gist of the present invention.

EXAMPLES

Group and Sample Analysis

The inventors of the present invention registered 118 consecutive patients suffering from stage 3, 4 and 5 CKD, who were not undergoing dialysis, from the First Department of Nephrology of the Rinku General Medical Center in a prospective study from August in 2005 to January in 2009. After fasting overnight, baseline blood sample were collected from the patients and plasma was prepared by placing in plastic tubes. Patients from whom inadequate blood samples were unable to be acquired were omitted in advance.

The study was approved by the ethics committee of the Rinku General Medical Center and was conducted on the basis of the Declaration of Helsinki.

Baseline inclusion criteria consisted of age of less than 90 years, absence of complications associated with malignant tumor and absence of infection. Patients from whom complete baseline data was unable to be acquired (n=2) or patients from whom adequate blood samples were unable to be acquired (n=4), and patients who began renal replacement therapy within 1 month after registration (n=4) were omitted from the study. The study was approved by the institutional ethics committee of the Rinku General Medical Center and the Osaka City General Hospital, and written informed consent to participate in the study was obtained from all patients. Renal function was evaluated from baseline data obtained during initial examination at this facility using estimated glomerular filtration rate (eGFR) based on an equation newly developed for Japanese.

That equation is as follows:

[Math. 1]

$$\mathrm{eGFR} = 194 \times \text{serum creatinine (SCr)}^{-1.094} \times \mathrm{age}^{-0.287}$$

(wherein, the units for age are years, the units for SCr are mg/dL, and the units for estimated glomerular filtration rate (eGFR) are mL/min/body surface area of $1.73m^2$).

A correction factor of 0.739 was multiplied by the value calculated from the formula for female patients.

Serum creatinine was measured by an in-house enzymatic method. Random urine samples (10 ml) were collected at the time of baseline determination followed by measurement of the ratios of urinary protein and creatinine. Other variables used when determining the baseline consisted of age, gender, diabetes as defined according to codes E10 to E14 of the 10th edition of the International Classification of Diseases (ICD), systolic blood pressure, diastolic blood pressure, hemoglobin level and the use of renin-angiotensin system inhibitors, β-blockers and calcium blockers. Baseline characteristics of the patients were as indicated below.

[Table 1]

Table 1 Patient Baseline Characteristics

| Characteristic | All Patients(n=108) |
|---|---|
| Age (years) | 65.3±10.9 |

(continued)

| Characteristic | All Patients(n=108) |
|---|---|
| Proportion of males (%) | 75.0 |
| eGFR (mL/min/1.73 $m^2$) | 21.0±12.4 |
| Mean blood pressure (mmHg) | 95.1±12.9 |
| Systolic blood pressure | 139.1±21.7 |
| Diastolic blood pressure | 73.2±11.7 |
| Hemoglobin (g/dL) | 11.0±1.9 |
| Urinary protein (g/gCre) | 2.8±3.8 |
| Patient origin (%) | |
| Diabetes | 30.6 |
| Chronic glomerulonephritis | 23.1 |
| Benign glomerulosclerosis | 35.2 |
| Other | 10.2 |
| Use of ACEi and/or ARB (%) | 68.8 |
| Use of β-blockers (%) | 32.4 |
| Use of calcium blockers (%) | 67.6 |

Values are indicated as the mean ± SD or as percent (%).

eGFR: Estimated glomerular filtration rate, ACEi: Angiotensin-converting enzyme inhibitor, ARB: Angiotensin II receptor blocker

In the present study, the initial endpoint defined as kidney outcome was the total of end-stage kidney disease (ESKD) requiring renal replacement therapy and all deaths. The patients underwent routine follow-up care on an outpatient basis. The data was gathered in the form of a source document in the end of 2011. Baseline and follow-up data were collected from hospital medical records and discharge summaries, outpatient records, interviews conducted at the time of initial examination and with the physician in charge of dialysis care, and death certificates. Endpoint was confirmed by at least two physicians. Patient follow-up data was able to be used accurately. This is because (i) this facility is a central hospital located in the southern part of Osaka prefecture and there are no other central hospitals located in this area, and (ii) there is a favorable working relationship with local physicians responsible for the initial examination and dialysis care.

Sample Preparation

Preparation of samples from human plasma was carried out in accordance with a modification of the procedure described in the Journal of Chromatography. B, Analytical technologies in the biomedical and life science, 966, 187-192 (2014). In short, this procedure consists of adding 20 volumes of methanol to the plasma, transferring a fixed amount (10 μl of supernatant obtained from the methanol homogenate) to a brown tube, and derivatizing with NBD (using 0.5 μl of plasma in the reaction). The solution is then dried under reduced pressure followed by the addition of 20 μl of 200 mM sodium borate buffer (pH 8.0) and 5 μl of a fluorescent labeling reagent (anhydrous MeCN containing 40 mM 4-fluoro-7-nitro-2,1,3-benzoxazole (NBD-F)) and heating for 2 minutes at 60°C. 0.1% aqueous TFA solution (75 μl) is then added and 2 μl of this reaction mixture is used in 2D-HPLC.

Measurement of Amino Acid Enantiomers by 2D-HPLC

Amino acid enantiomers were assayed using a Micro 2D-HPLC platform as described in J. Chromatogr. A: 1217, 1056-1062 (2010) and the Journal of Chromatography, B: Analytical technologies in the biomedical and life sciences, 877, 2506-2512 (2009). In short, NBD derivatives of the amino acids were eluted by gradient elution using an aqueous mobile phase containing MeCN, THF and TFA using a reverse phase column (Monolithic ODS Column, 0.53 mm i.d. × 100 mm, Shiseido Japan Co., Ltd.). Target amino acid fractions were recovered automatically using a multi-loop valve in order to separate and measure the D- and L-forms followed by supplying to an enantiomer selection column (KSAACSP-001S or Sumichiral oA-3200, 1.5 mm i.d. × 250 mm, self-filling, materials acquired from Shiseido Japan and Sumika Chemical Analysis Services). In the case of measuring Ile and Thr having four types of stereoisomers, the L- and D-forms along with diastereoisomers (L-allo form and D-allo form) were separated by the first dimensional reverse phase mode (and these diastereoisomers are separated in the reverse phase mode). Next, the enantiomers (L- and D-forms and L-allo- and D-allo forms) were separated two-dimensionally with an enantiomer selection column. The mobile phase consisted of a mixed solution of MeOH and MeCN containing citric acid or formic acid, and fluorescence of the NBD-amino acids was excited at 470 nm and detected at 530 nm. All assay data was acquired by fluorescence detection. The actual presence of D-amino acids in the biological matrix was confirmed using HPLC-MS/MS.

Statistical Processing

Determination of diabetes was made by two physicians based on codes E10 to E14 of the 10th edition of the International Classification of Diseases (ICD-10). When each of the separated chiral amino acids was grouped between diabetes patients and non-diabetes patients, significantly lower values were demonstrated for D-Asp, D-Pro and L-Gle, while significantly higher values were demonstrated for L-Ile (Figs. 2A to 2D). Next, in order to investigate the diagnostic specificity of each of these chiral amino acids with respect to diabetes, ROC curves were rendered for the present cohorts (Figs. 1A to 1D). When cutoff values for determining diabetes were determined on the basis of these ROC curves, the cutoff values consisted of 0.1 μg/ml in the case of D-aspartic acid, 2.5 μg/ml in the case of D-proline, 665 μg/ml in the case of L-glutamine and 49.3 μg/ml in the case of L-isoleucine.

Next, the correlation between age and the amount of each separated chiral amino acid was investigated. When age and the amount of each chiral amino acid was represented with a scatter diagram followed by calculating the correlation coefficients thereof, a correlation was observed between age and the amount of chiral amino acid in blood samples for D-Ala, D-Pro, D-alloIle, D-Leu, L-Ile and L-Ser ($p<0.05$) (Fig. 3A to 3F(1)). Next, the patients were divided into an age 70 or older group and an under age 70 group and ROC curves were rendered in order to investigate determinant specificity between age and each chiral amino acid (Fig. 3A to 3F(2)). In addition, the ratio with the corresponding isomer (namely, L-Ala and L-Pro) and D/L% were calculated for D-Ala and D-Pro, and ROC curves were also rendered to investigate the determinant specificity of age in the same groups with respect to D/L% (Figs. 3A to 3B(3)). When cutoff values were determined in order to determine subjects older than age 70 based on the ROC curves, the cutoff values consisted of 4.7 μg/ml or more in the case of D-Ala, 2.5 μg/ml or more in the case of D-Pro, 0.1 μg/ml or more in the case of D-alloIle, 0.5 (μg/ml or more in the case D-Leu, 134.6 or less in the case of L-Ser and 58.6 or less in the case of L-Ile. The cutoff value for D/L% in the case of Ala was 1.3 or more and that in the case of Pro was 1.1 or more.

Next, the correlation between estimated glomerular filtration rate (eGFR) and the amount of each separated chiral amino acid was investigated. When the value of eGFR and the amount of each chiral amino acid were represented with a scatter diagram followed by calculating the correlation coefficients thereof, a correlation was observed between the value of eGFR and the amount of chiral amino acid in blood samples for D-asparagine, D-serine, D-aspartic acid, D-allothreonine, D-alanine, D-proline, D-leucine, L-histidine, L-serine, L-aspartic acid, L-alanine, L-isoleucine, L-phenylalanine, L-tryptophan, L-lysine and L-tyrosine (Figs. 4A to 4L(1) to (3)).

**Claims**

1. A blood sample analysis method for determining diabetes in a subject, comprising:

   a step for measuring the amount of at least one type of amino acid selected from the group consisting of D-aspartic acid, D-proline, L-glutamine and L-isoleucine, and
   a step for determining diabetes by comparing the measured amount of the at least one type of amino acid with a predetermined cutoff value.

2. The analysis method according to claim 1, wherein the cutoff value is determined based on an ROC curve.

3. The analysis method according to claim 1 or 2, wherein the cutoff value is 0.1 μg/ml in the case of D-aspartic acid, 2.5 μg/ml in the case of D-proline, 665 (μg/ml in the case of L-glutamine and 49.3 μg/ml in the case of L-isoleucine.

4. The analysis method according to any of claims 1 to 3, wherein the subject is a subject suffering from kidney disease.

5. A blood analysis system for determining diabetes in a subject, comprising a storage unit, an input unit, a data processing unit and an output unit, and comprising the following step:

inputting a cutoff value of at least one type of chiral amino acid in the blood for determining diabetes and pathology information on diabetes from the input unit and storing in the storage unit,
inputting a measured value of at least one type of chiral amino acid in a blood sample of the subject from the input unit and storing in the storage unit,
comparing the stored measured value of the amino acid with a stored cutoff value by the data processing unit to determine pathology information on diabetes of the subject, and
outputting the diabetes pathology information to the output unit;

wherein the chiral amino acid present in the blood used to determine diabetes is at least one type of amino acid selected from the group consisting of D-aspartic acid, D-proline, D-glutamine and L-isoleucine.

**6.** The blood analysis system according to claim 5, which further comprises an analysis/measurement unit, and the analysis/measurement unit determining a measured value of a chiral amino acid in the blood of a blood sample of a subject by separating and assaying the chiral amino acid, and inputting the measured value instead of the input unit or via the input unit.

**7.** The blood analysis system according to claim 5 or 6, wherein the cutoff value is determined based on an ROC curve.

**8.** The blood analysis system according to any of claims 5 to 7, wherein the cutoff value is 0.1 μg/ml in the case of D-aspartic acid, 2.5 μg/ml in the case of D-proline, 665 μg/ml in the case of L-glutamine and 49.3 μg/ml in the case of L-isoleucine.

**9.** A method for estimating age in a subject, comprising:

a step for measuring the amount of at least one type of amino acid selected from the group consisting of D-alanine, D-leucine, D-allo-isoleucine, D-proline, L-serine and L-isoleucine in a blood sample, and
a step for determining age based on the measured amount of the at least one type of amino acid and a predetermined age and amino acid levels.

**10.** The estimation method according to claim 9, wherein in the step for determining age, age is determined based on a cutoff value.

**11.** The estimation method according to claim 10, wherein the cutoff value is determined based on an ROC curve and the cutoff value for estimating an age of 70 years or older is 4.7 μg/ml or more in the case of D-alanine, 2.5 μg/ml or more in the case of D-proline, 0.1 μg/ml or more in the case of D-alloisoleucine, 0.50 or more in the case of D-leucine, 134.6 or less in the case of L-serine and 58.6 or less in the case of L-isoleucine.

**12.** The estimation method according to claim 9, wherein in the step for determining age, age is determined based on a predetermined regression curve.

**13.** A system for determining the estimated age of a subject, comprising a storage unit, an input unit, a data processing unit and an output unit; comprising carrying out the following step:,

inputting the amount of at least one type of chiral amino acid in the blood for determining age and a regression curve or cutoff value for age from the input unit and stored in the storage unit,
inputting a measured value of the amount of at least one type of chiral amino acid in the blood sample of the subject from the input unit and stored in the storage unit,
determining estimated age of the subject by the data processing unit based on the stored measured value of the amount of the amino acid and the regression curve or cutoff value, and
outputting the estimated age to the output unit;,
wherein the chiral amino acid in the blood used to determine age is at least one type of amino acid selected from the group consisting of D-alanine, D-leucine, D-alloisoleucine, D-proline, L-serine and L-isoleucine.

FIG. 1-A

D-Asp

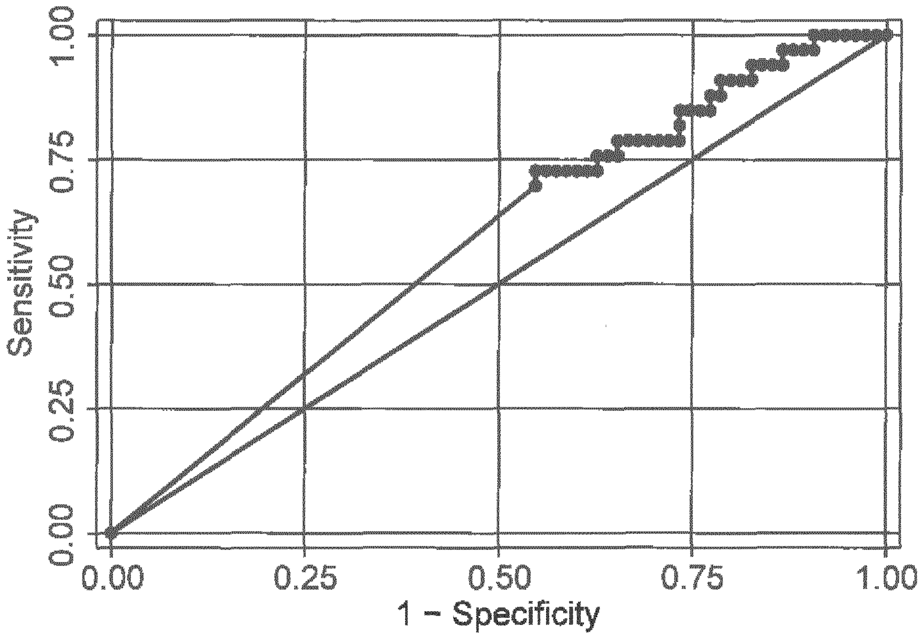


Area under ROC curve = 0.5836

FIG. 1-B

D-Pro

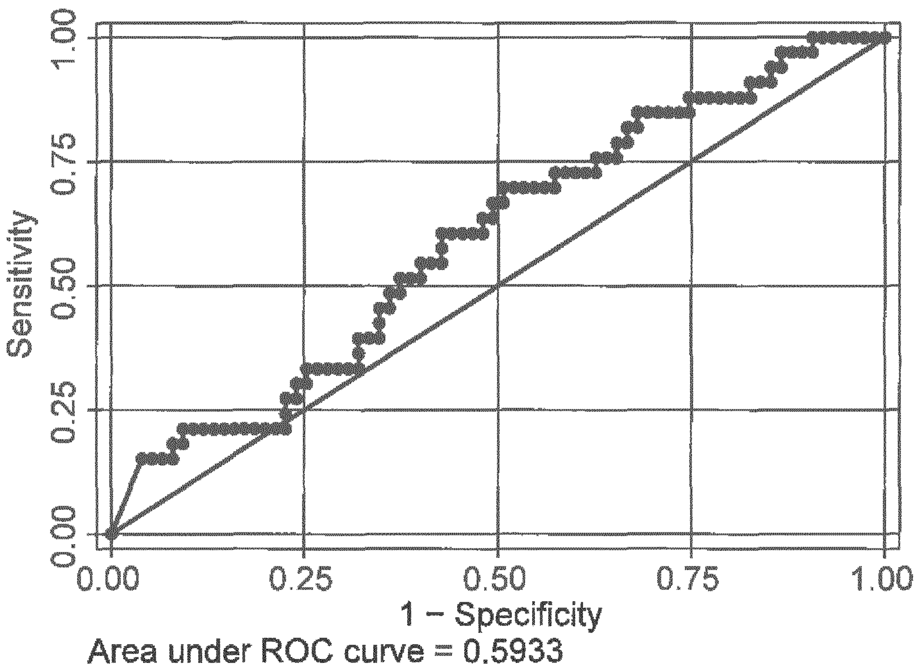


Area under ROC curve = 0.5933

FIG. 1-C

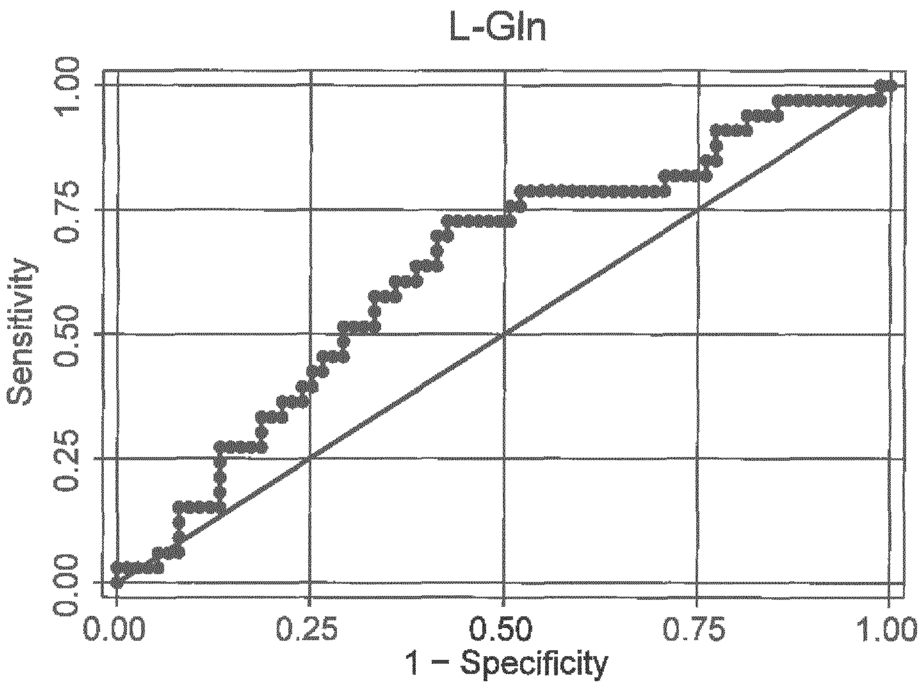


Area under ROC curve = 0.6327

FIG. 1-D

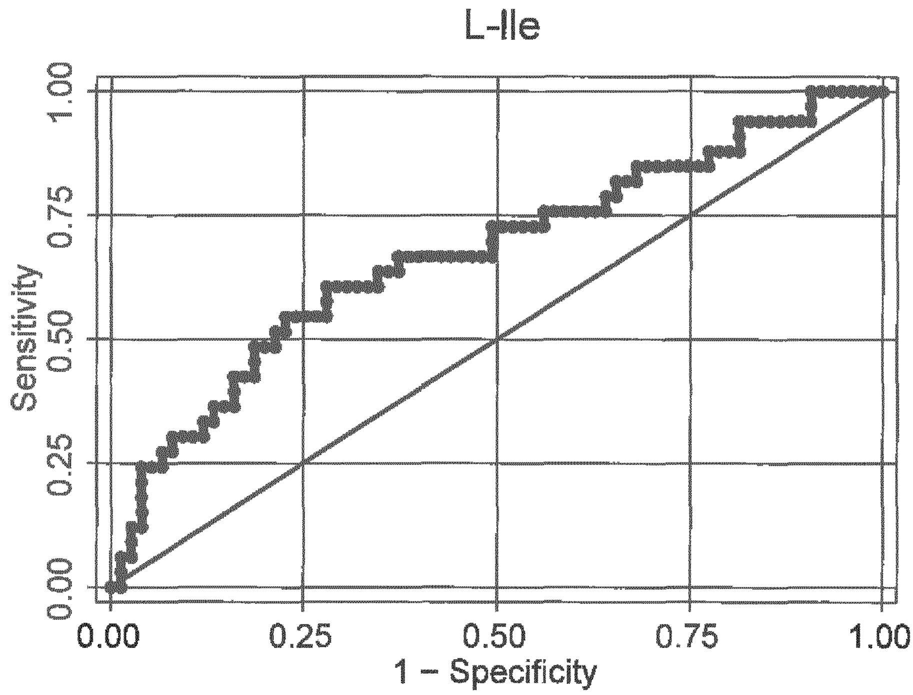


Area under ROC curve = 0.6731

FIG. 2-A

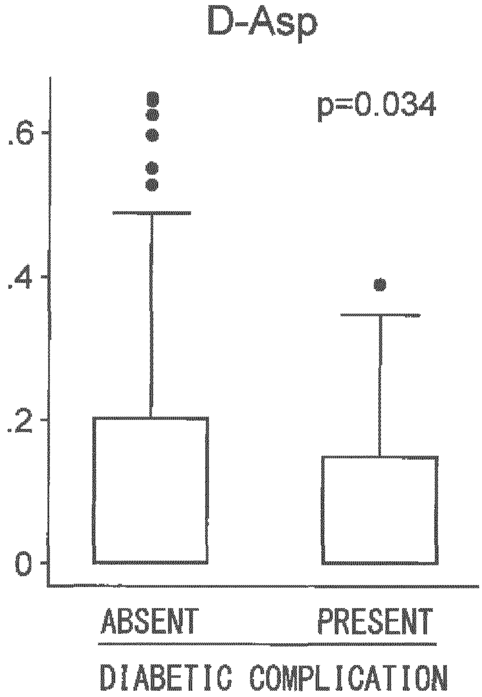
D-Asp
p=0.034
.6
.4
.2
0
ABSENT
PRESENT
DIABETIC COMPLICATION

FIG. 2-B

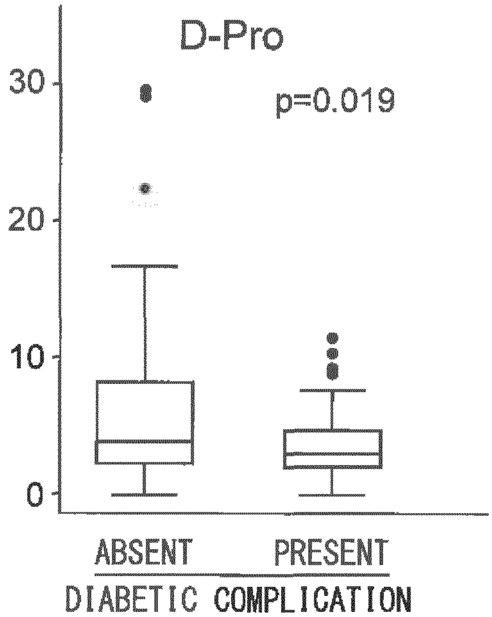
D-Pro
p=0.019
30
20
10
0
ABSENT
PRESENT
DIABETIC COMPLICATION

FIG. 2-C

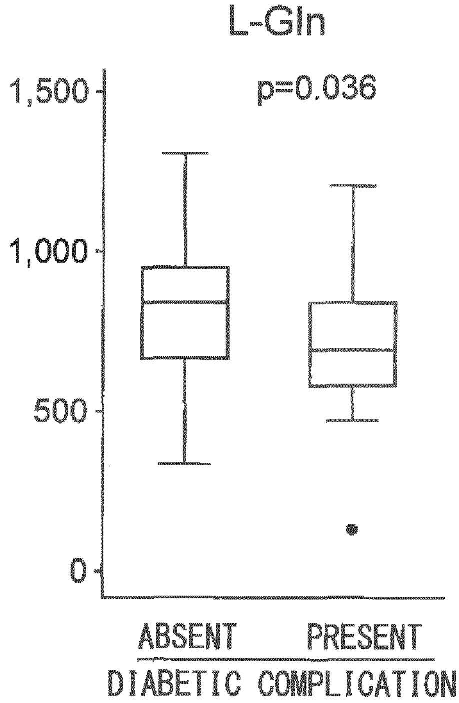
L-Gln
p=0.036
1,500
1,000
500
0
ABSENT
PRESENT
DIABETIC COMPLICATION

FIG. 2-D

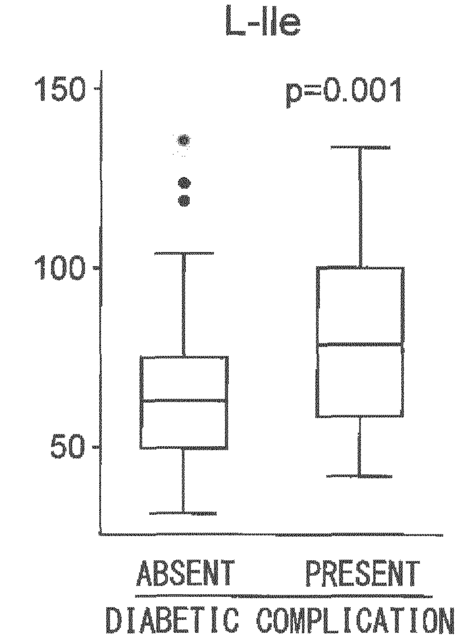
L-Ile
p=0.001
150
100
50
ABSENT
PRESENT
DIABETIC COMPLICATION

FIG. 3-A

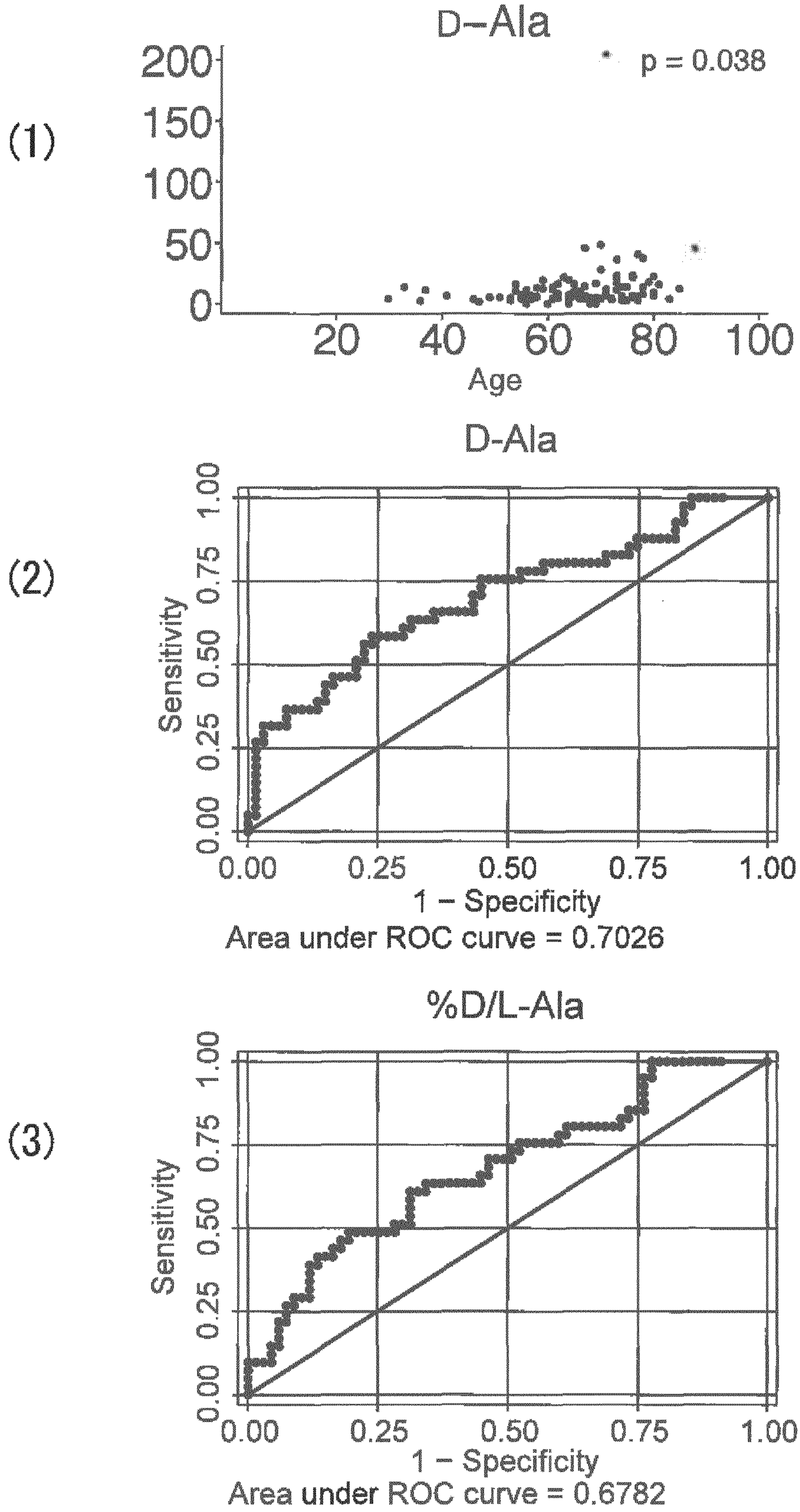
(1)
D-Ala
p = 0.038
200
150
100
50
0
20
40
60
80
100
Age
(2)
D-Ala
Sensitivity
0.00
0.25
0.50
0.75
1.00
1 − Specificity
Area under ROC curve = 0.7026
(3)
%D/L-Ala
Sensitivity
0.00
0.25
0.50
0.75
1.00
1 − Specificity
Area under ROC curve = 0.6782

FIG. 3-B

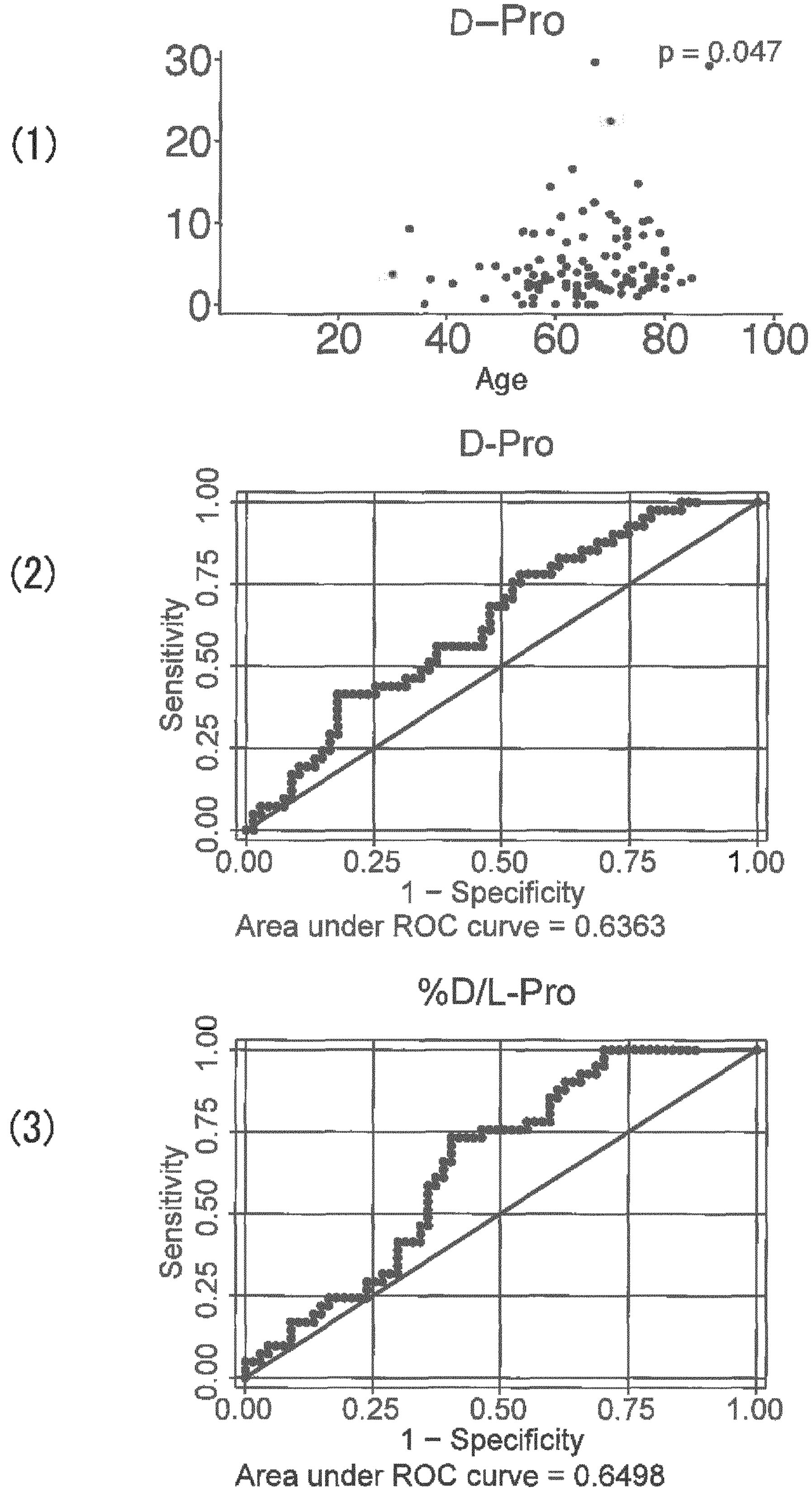
(1)
D-Pro
p = 0.047
30
20
10
0
20
40
60
80
100
Age
(2)
D-Pro
Sensitivity
0.00
0.25
0.50
0.75
1.00
1 − Specificity
Area under ROC curve = 0.6363
(3)
%D/L-Pro
Sensitivity
0.00
0.25
0.50
0.75
1.00
1 − Specificity
Area under ROC curve = 0.6498

FIG. 3-C

(1)

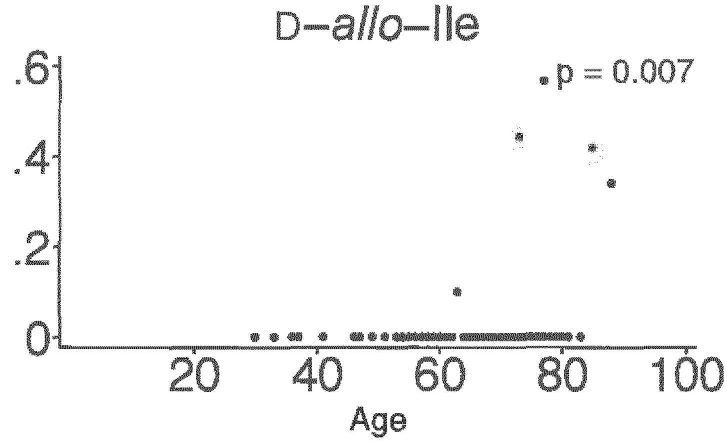
D-allo-Ile
p = 0.007
.6
.4
.2
0
20
40
60
80
100
Age

(2)

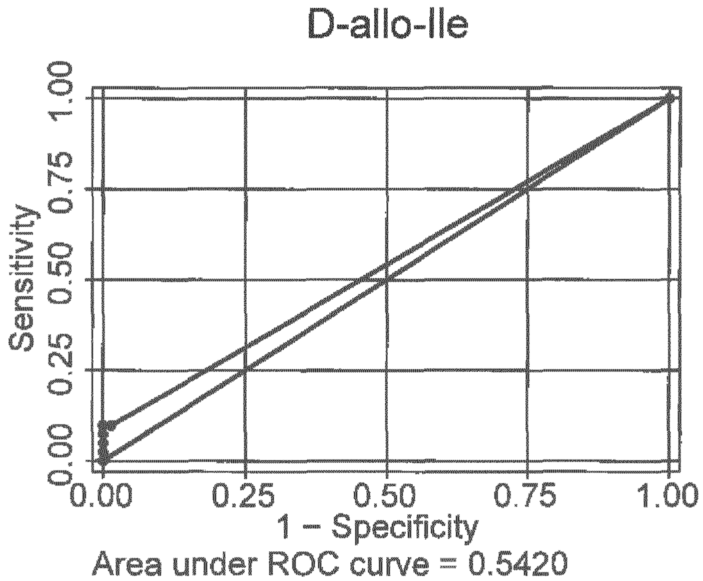
D-allo-Ile
Sensitivity
0.00
0.25
0.50
0.75
1.00
0.00
0.25
0.50
0.75
1.00
1 − Specificity
Area under ROC curve = 0.5420

FIG. 3-D

(1)

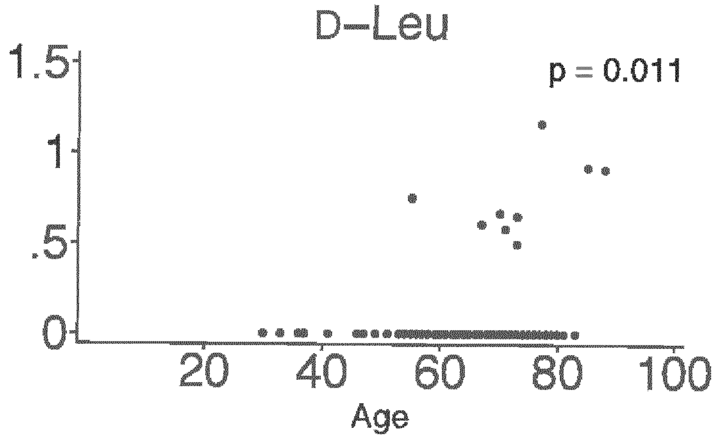
D-Leu
p = 0.011
1.5
1
.5
0
20
40
60
80
100
Age

(2)

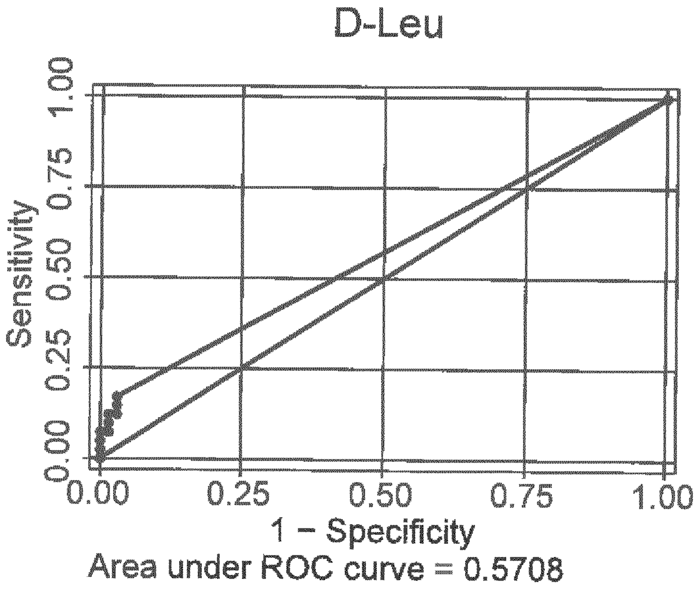
D-Leu
Sensitivity
0.00
0.25
0.50
0.75
1.00
1 - Specificity
Area under ROC curve = 0.5708

FIG. 3-E

(1)

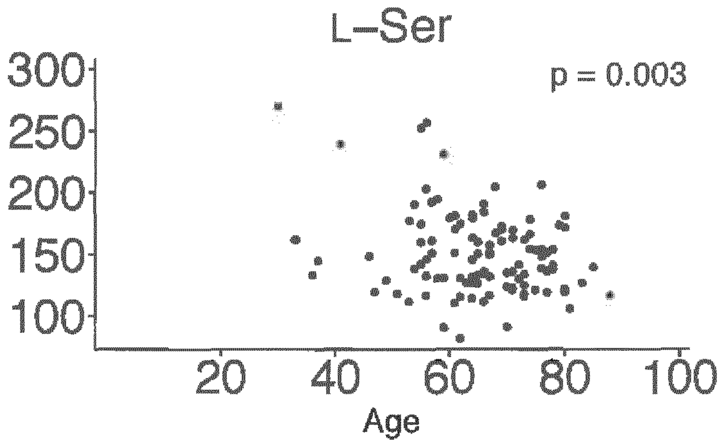
L–Ser
p = 0.003
300
250
200
150
100
20
40
60
80
100
Age

(2)

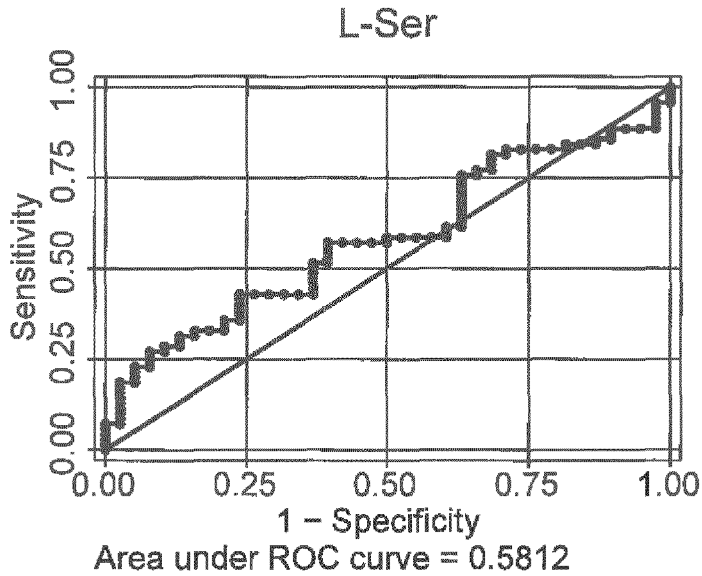
L-Ser
Sensitivity
0.00
0.25
0.50
0.75
1.00
0.00
0.25
0.50
0.75
1.00
1 − Specificity
Area under ROC curve = 0.5812

FIG. 3-F

(1)

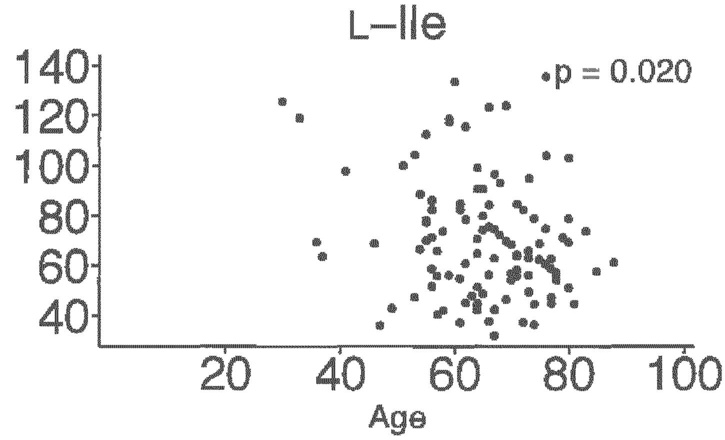
L-Ile
p = 0.020
140
120
100
80
60
40
20
40
60
80
100
Age

(2)

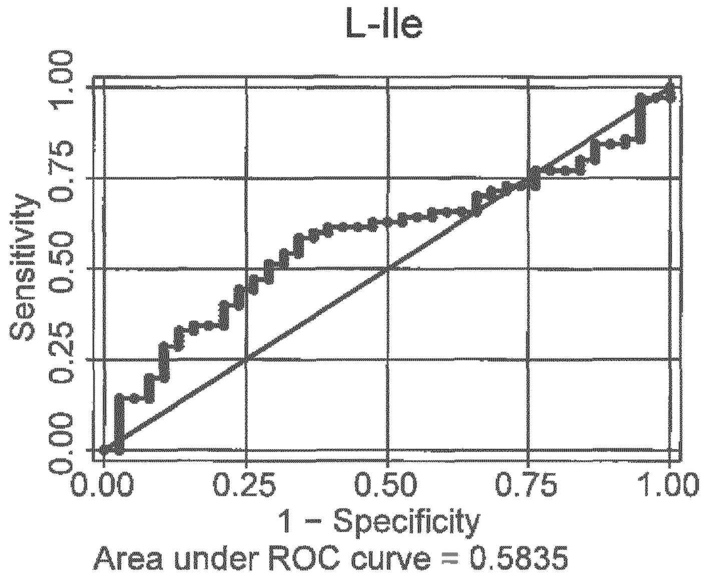
L-Ile
Sensitivity
0.00
0.25
0.50
0.75
1.00
1 - Specificity
Area under ROC curve = 0.5835

FIG. 4-A

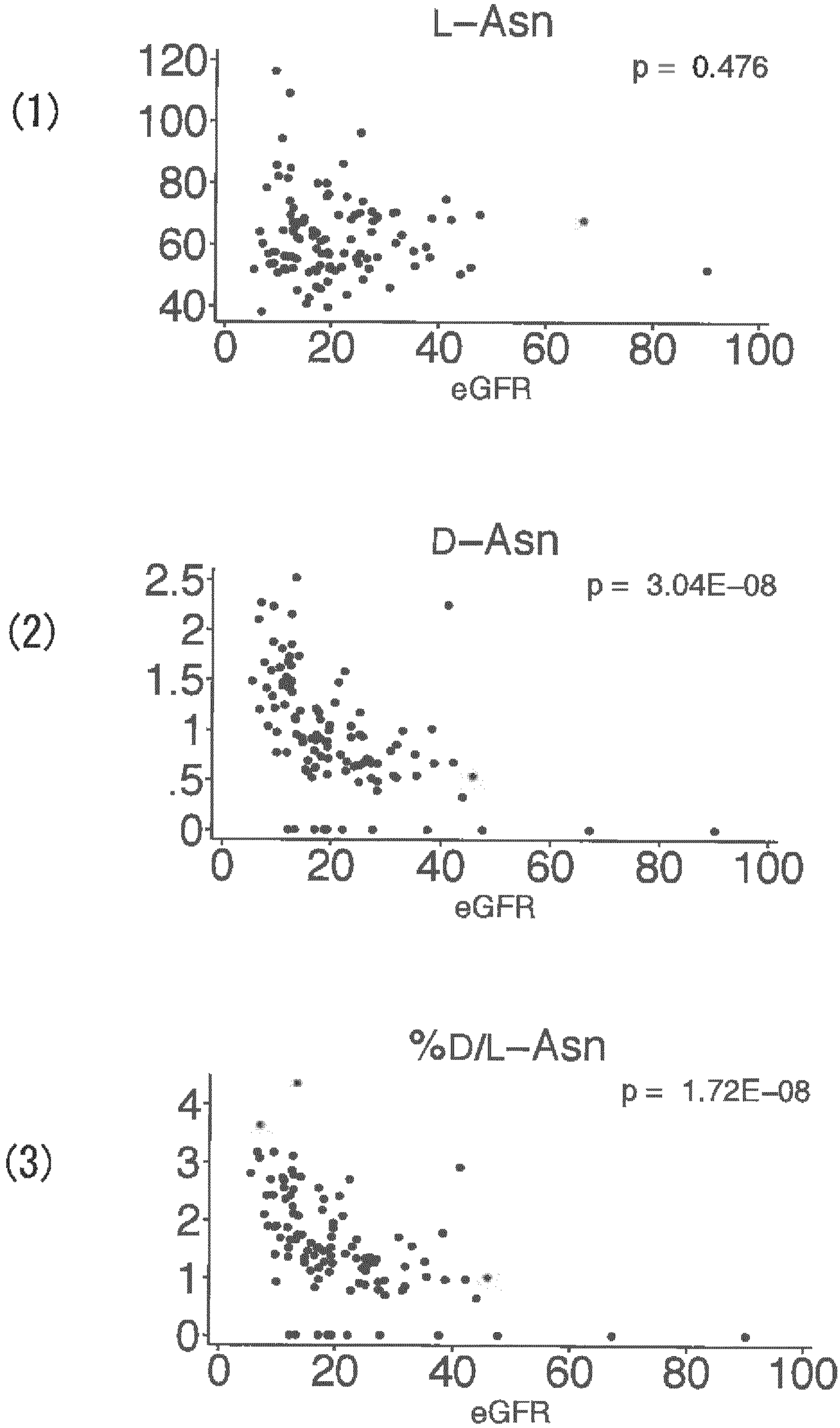
L-Asn
(1)
p = 0.476
120
100
80
60
40
0
20
40
60
80
100
eGFR
D-Asn
(2)
p = 3.04E-08
2.5
2
1.5
1
.5
0
0
20
40
60
80
100
eGFR
%D/L-Asn
(3)
p = 1.72E-08
4
3
2
1
0
0
20
40
60
80
100
eGFR

FIG. 4-B

(1)

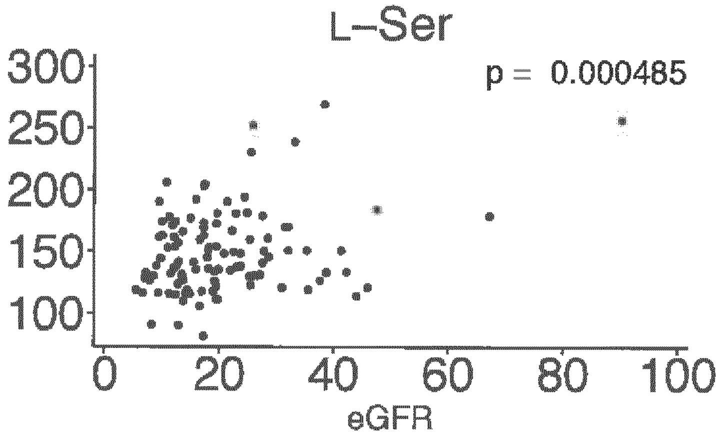
L-Ser
p = 0.000485
300
250
200
150
100
0
20
40
60
80
100
eGFR

(2)

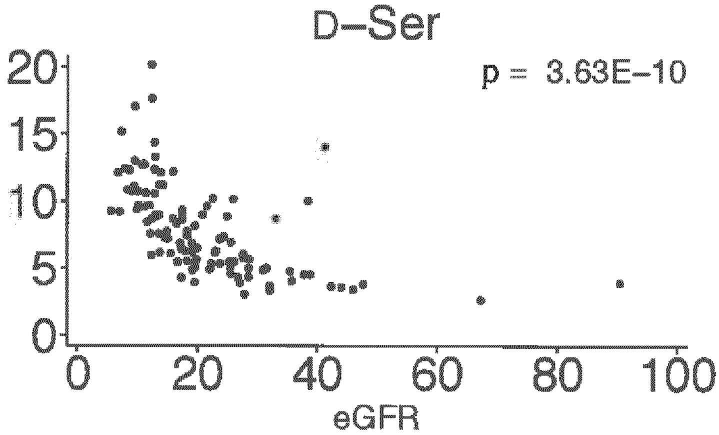
D-Ser
p = 3.63E-10
20
15
10
5
0
0
20
40
60
80
100
eGFR

(3)

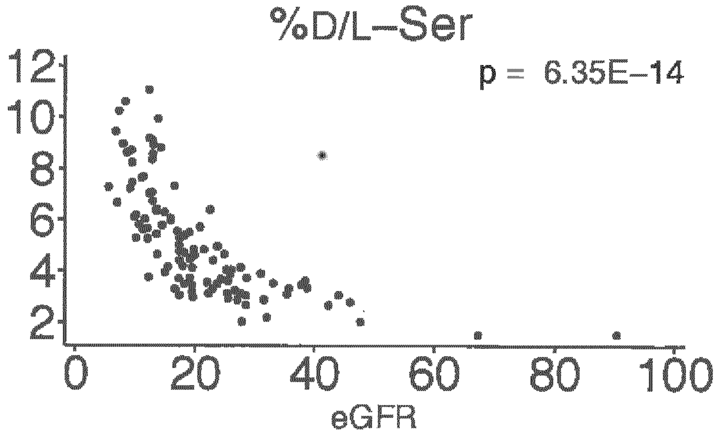
%D/L-Ser
p = 6.35E-14
12
10
8
6
4
2
0
20
40
60
80
100
eGFR

FIG. 4-C

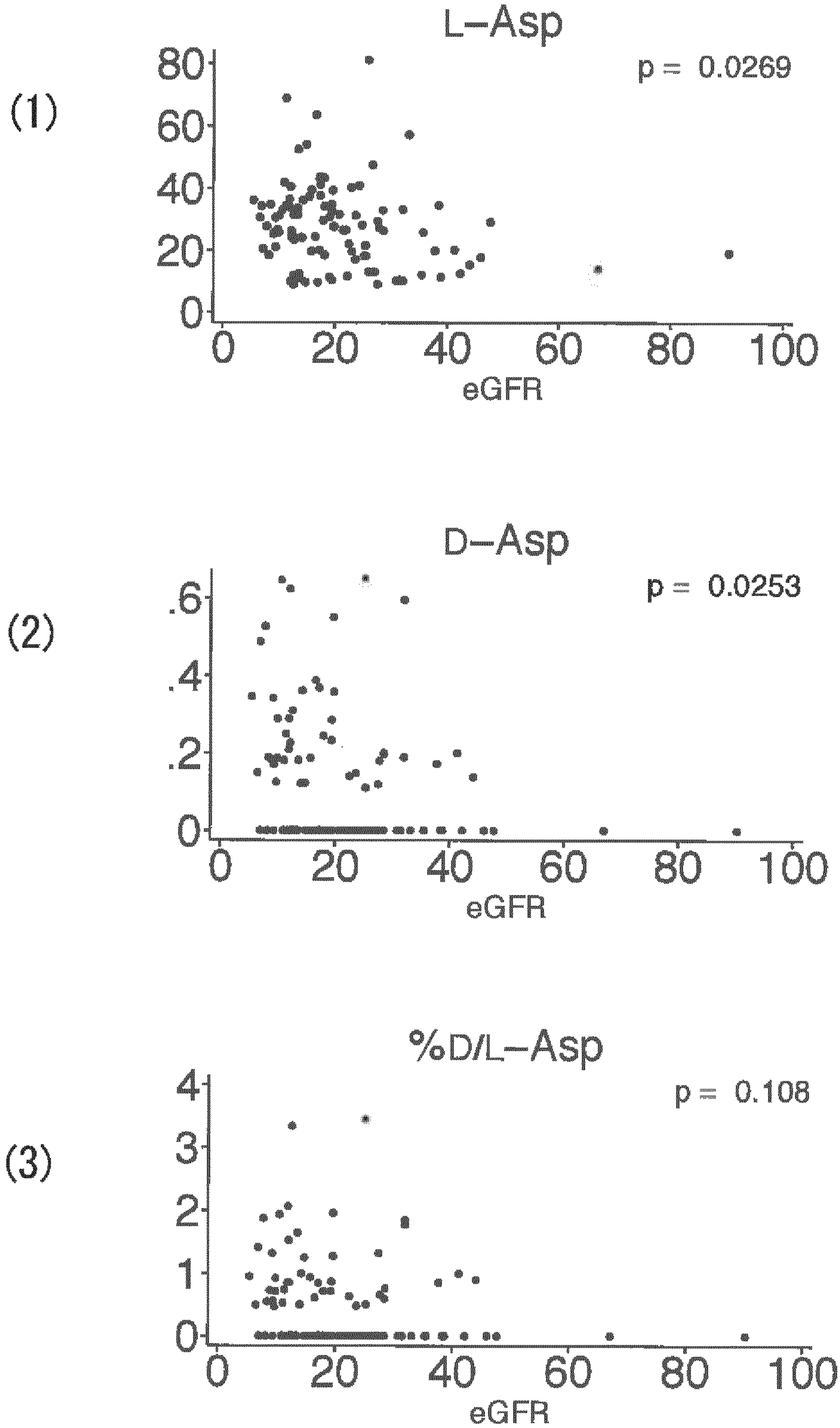
(1)
L−Asp
p = 0.0269
80
60
40
20
0
0 20 40 60 80 100
eGFR
(2)
D−Asp
p = 0.0253
.6
.4
.2
0
0 20 40 60 80 100
eGFR
(3)
%D/L−Asp
p = 0.108
4
3
2
1
0
0 20 40 60 80 100
eGFR

FIG. 4-D

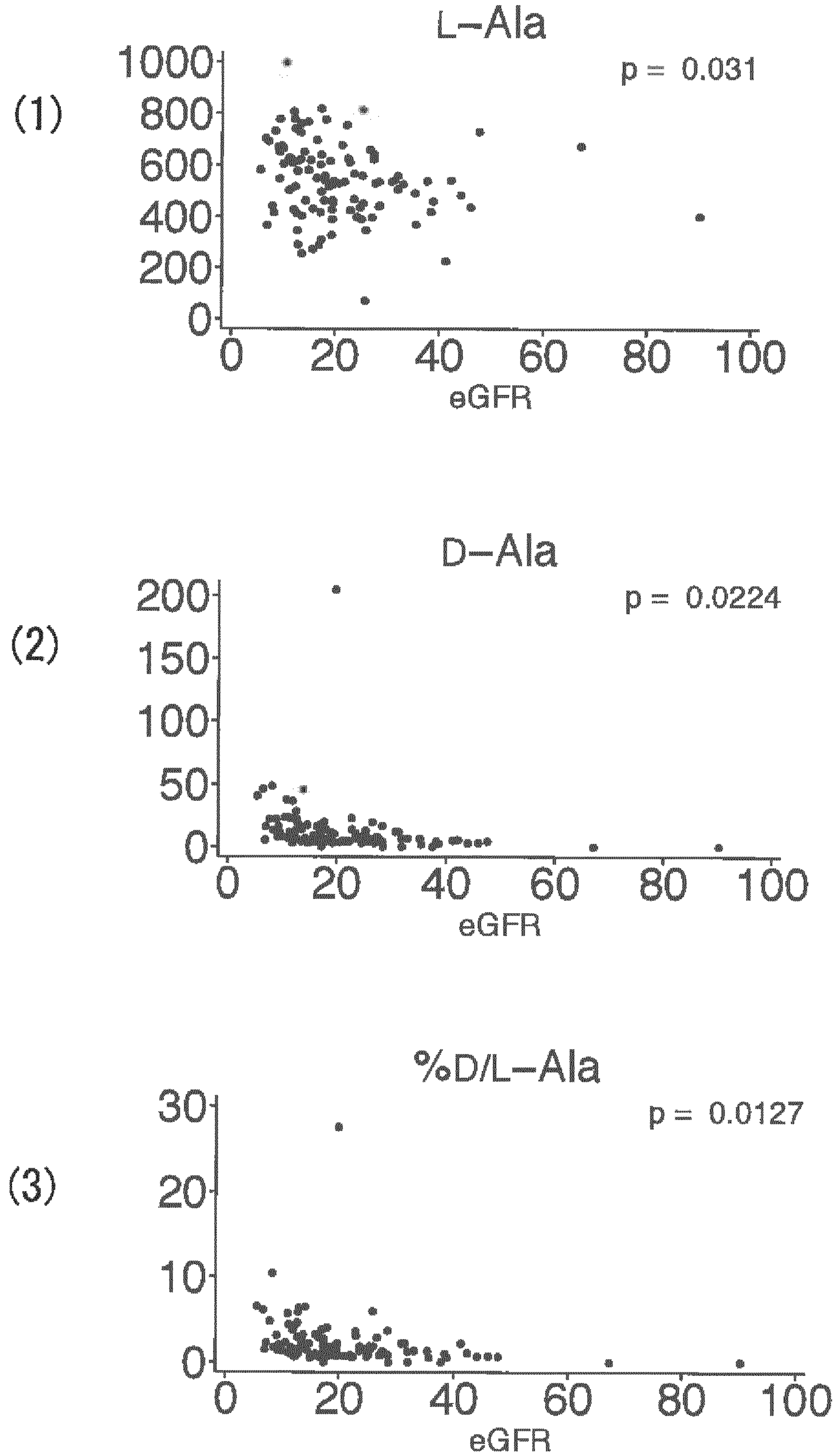
L-Ala
(1)
p = 0.031
1000
800
600
400
200
0
0 20 40 60 80 100
eGFR
D-Ala
(2)
p = 0.0224
200
150
100
50
0
0 20 40 60 80 100
eGFR
%D/L-Ala
(3)
p = 0.0127
30
20
10
0
0 20 40 60 80 100
eGFR

FIG. 4-E

(1)

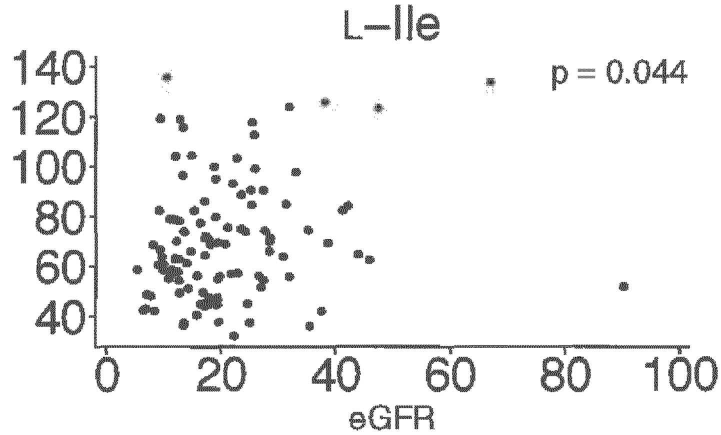
L-Ile
p = 0.044
140
120
100
80
60
40
0
20
40
60
80
100
eGFR

(2)

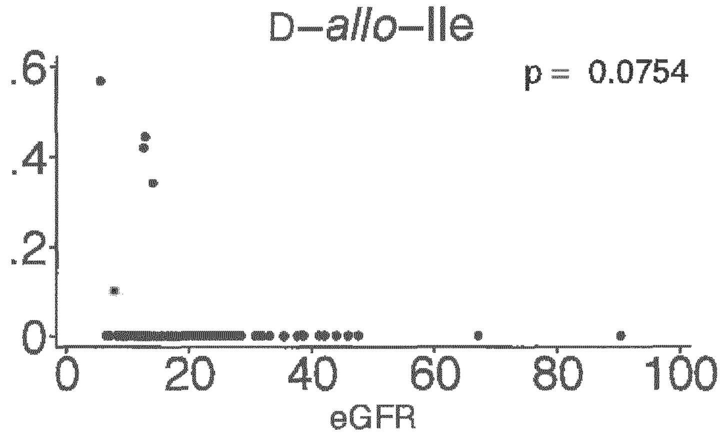
D-*allo*-Ile
p = 0.0754
.6
.4
.2
0
0
20
40
60
80
100
eGFR

FIG. 4-F

(1)

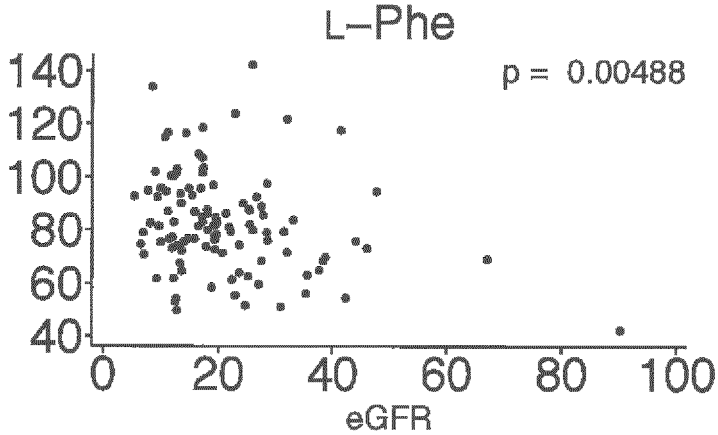
L-Phe
p = 0.00488
140
120
100
80
60
40
0
20
40
60
80
100
eGFR

(2)

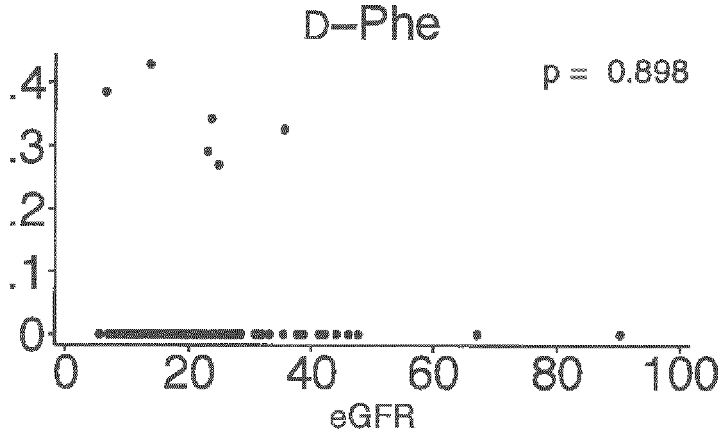
D-Phe
p = 0.898
.4
.3
.2
.1
0
0
20
40
60
80
100
eGFR

(3)

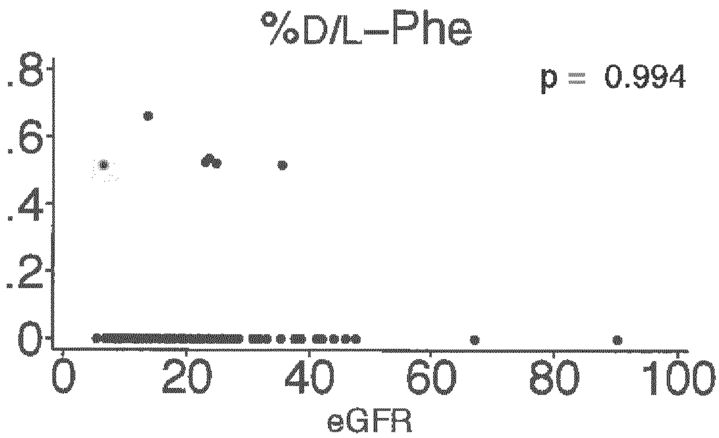
%D/L-Phe
p = 0.994
.8
.6
.4
.2
0
0
20
40
60
80
100
eGFR

FIG. 4-G

(1)

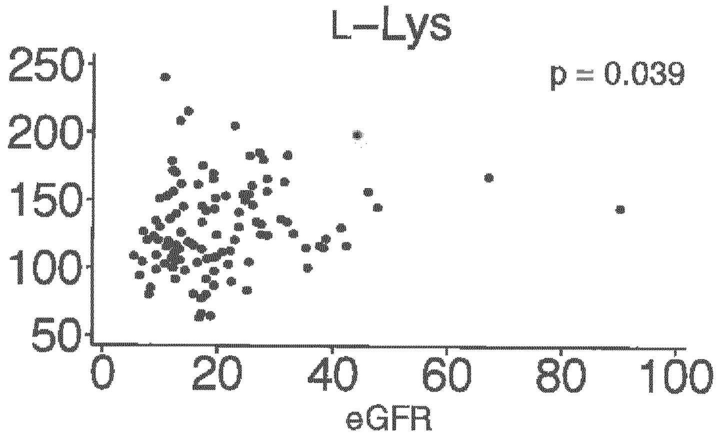
L-Lys
p = 0.039
250
200
150
100
50
0
20
40
60
80
100
eGFR

(2)

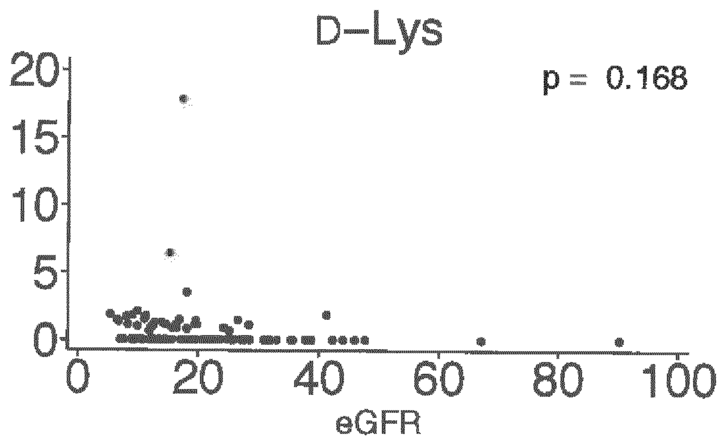
D-Lys
p = 0.168
20
15
10
5
0
0
20
40
60
80
100
eGFR

(3)

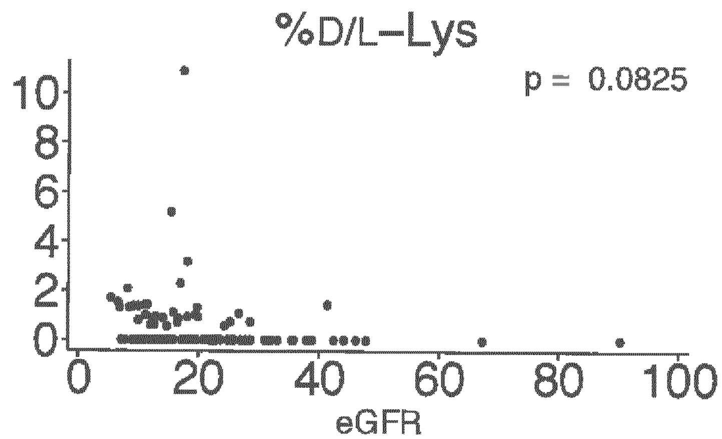
%D/L-Lys
p = 0.0825
10
8
6
4
2
0
0
20
40
60
80
100
eGFR

FIG. 4-H

(1)

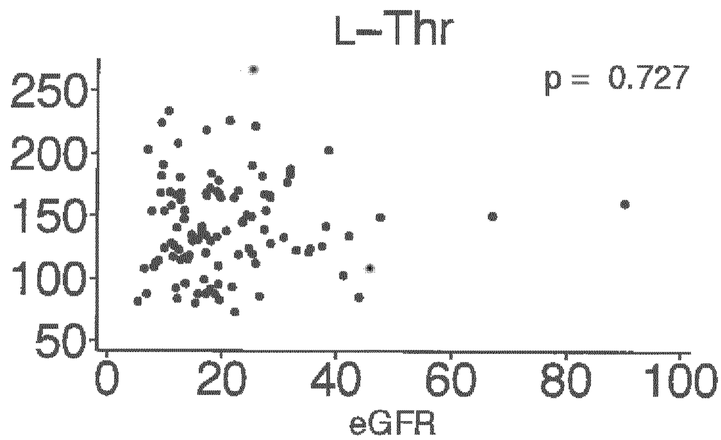
L-Thr
p = 0.727
250
200
150
100
50
0
20
40
60
80
100
eGFR

(2)

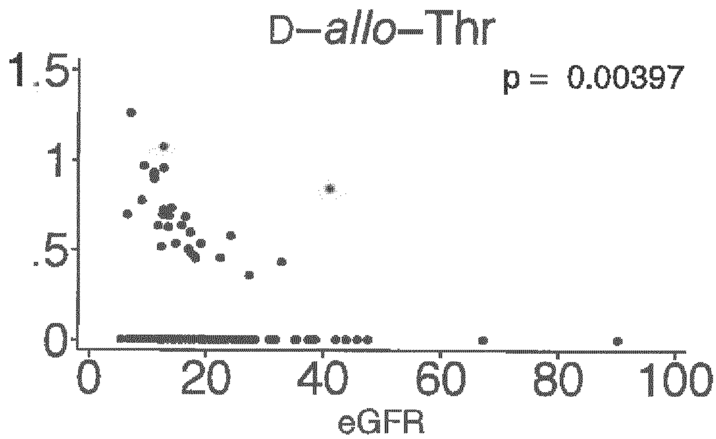
D-*allo*-Thr
p = 0.00397
1.5
1
.5
0
0
20
40
60
80
100
eGFR

FIG. 4-I

(1)

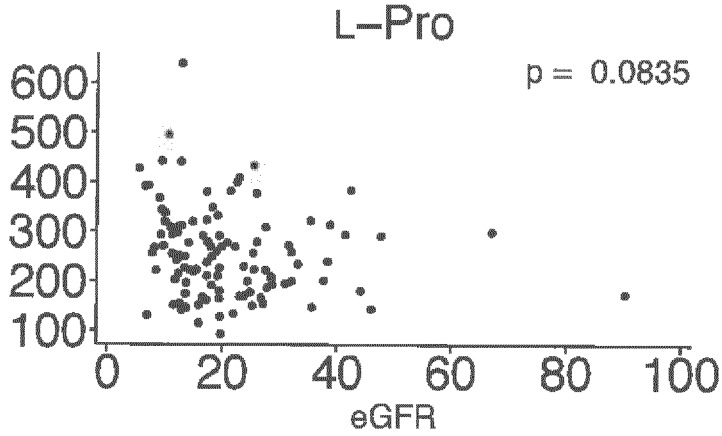
L-Pro
p = 0.0835
600
500
400
300
200
100
0
20
40
60
80
100
eGFR

(2)

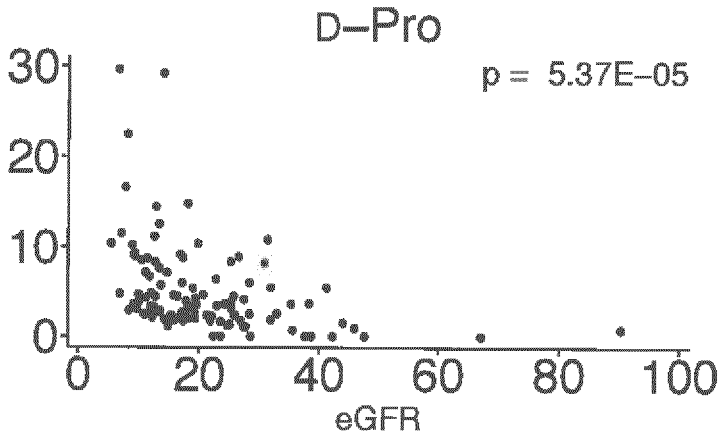
D-Pro
p = 5.37E-05
30
20
10
0
0
20
40
60
80
100
eGFR

(3)

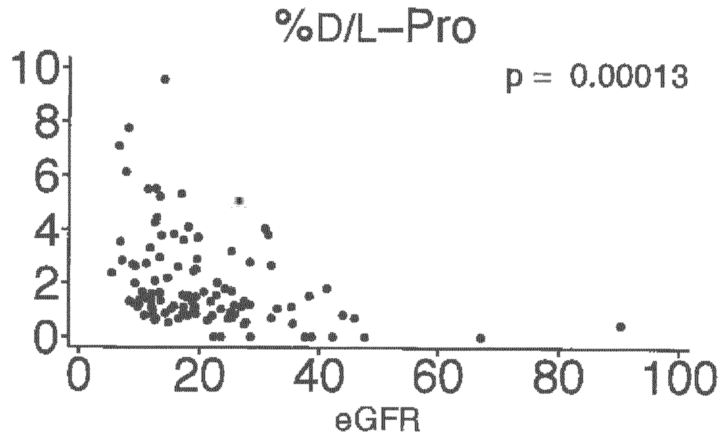
%D/L-Pro
p = 0.00013
10
8
6
4
2
0
0
20
40
60
80
100
eGFR

FIG. 4-J

(1)

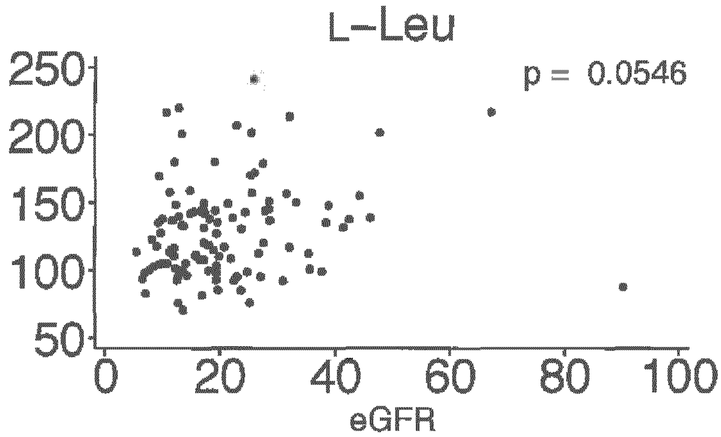
L-Leu
p = 0.0546
250
200
150
100
50
0
20
40
60
80
100
eGFR

(2)

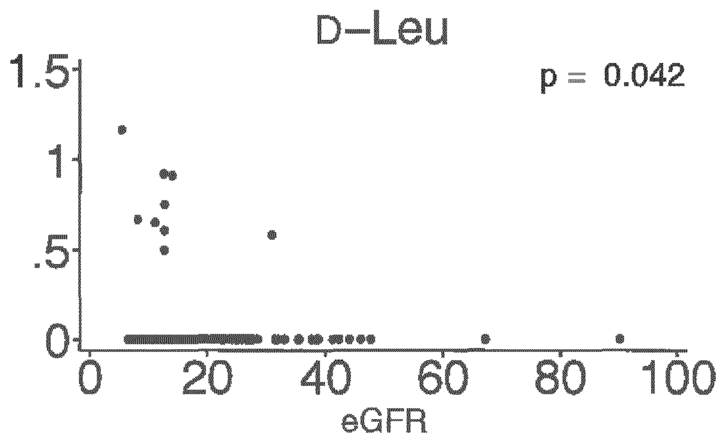
D-Leu
p = 0.042
1.5
1
.5
0
0
20
40
60
80
100
eGFR

(3)

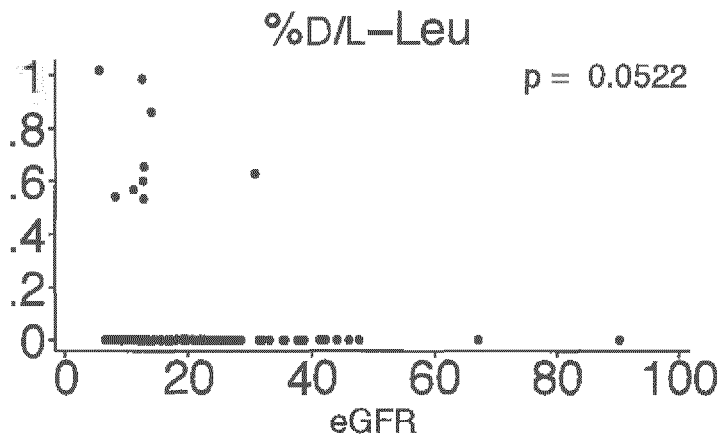
%D/L-Leu
p = 0.0522
1
.8
.6
.4
.2
0
0
20
40
60
80
100
eGFR

FIG. 4-K

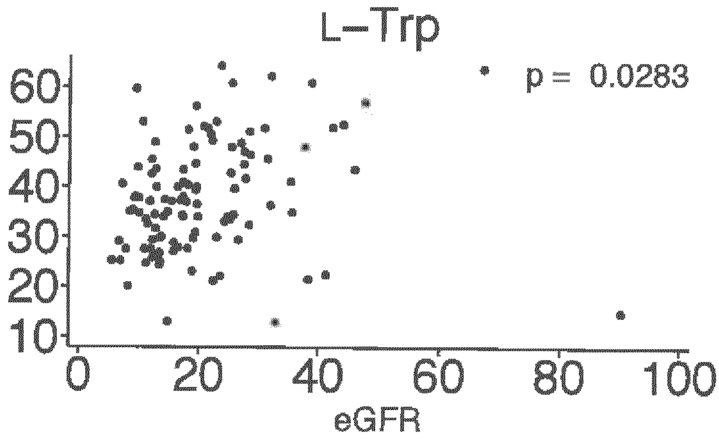
L-Trp
p = 0.0283
60
50
40
30
20
10
0
20
40
60
80
100
eGFR

FIG. 4-L

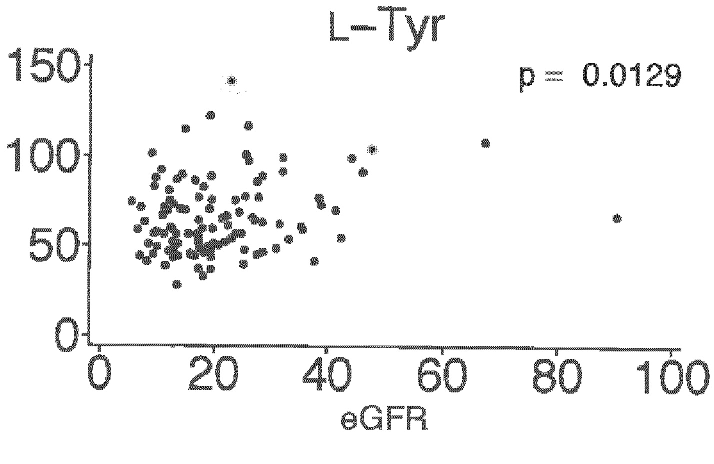
L-Tyr
p = 0.0129
150
100
50
0
0
20
40
60
80
100
eGFR

FIG. 4-M

(1)

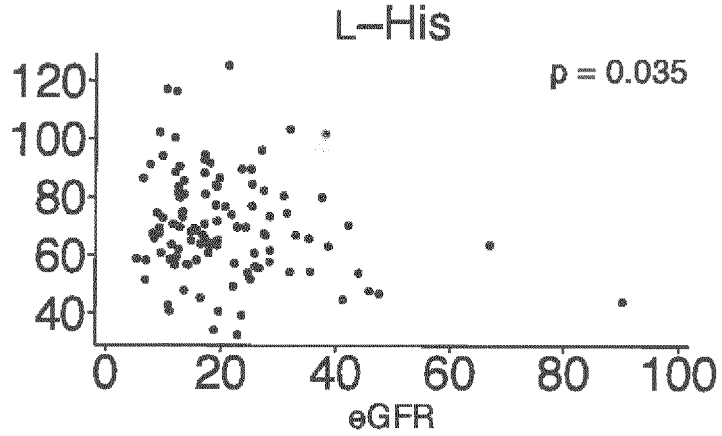
L–His
p = 0.035
120
100
80
60
40
0
20
40
60
80
100
eGFR

(2)

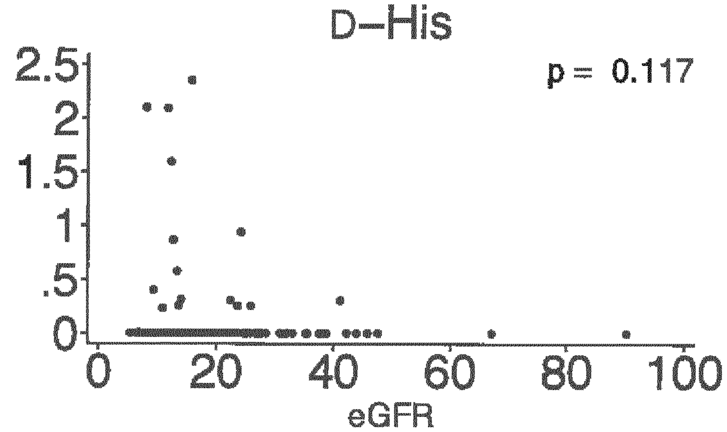
D–His
p = 0.117
2.5
2
1.5
1
.5
0
0
20
40
60
80
100
eGFR

(3)

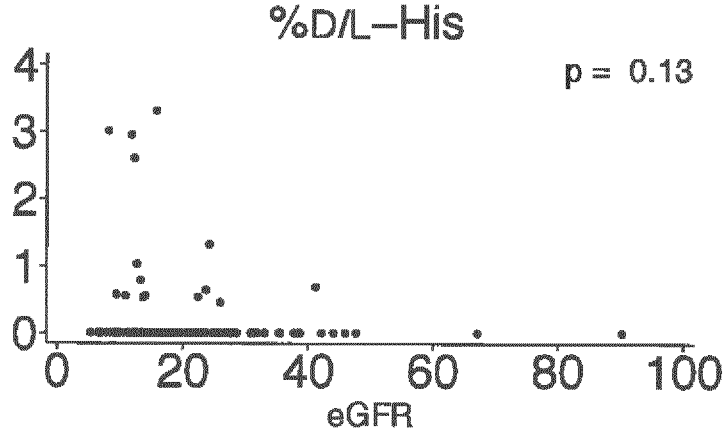
%D/L–His
p = 0.13
4
3
2
1
0
0
20
40
60
80
100
eGFR

FIG. 5

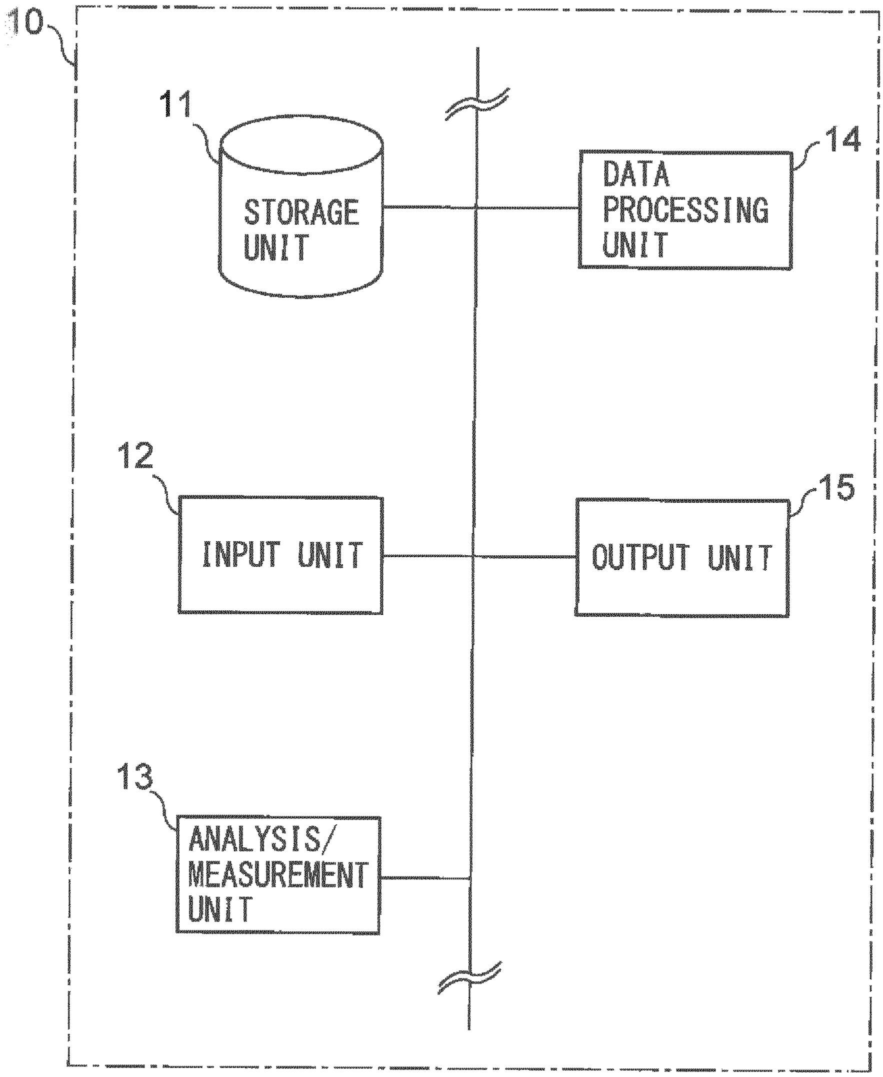
10
11
STORAGE UNIT
14
DATA PROCESSING UNIT
12
INPUT UNIT
15
OUTPUT UNIT
13
ANALYSIS/ MEASUREMENT UNIT

FIG. 6

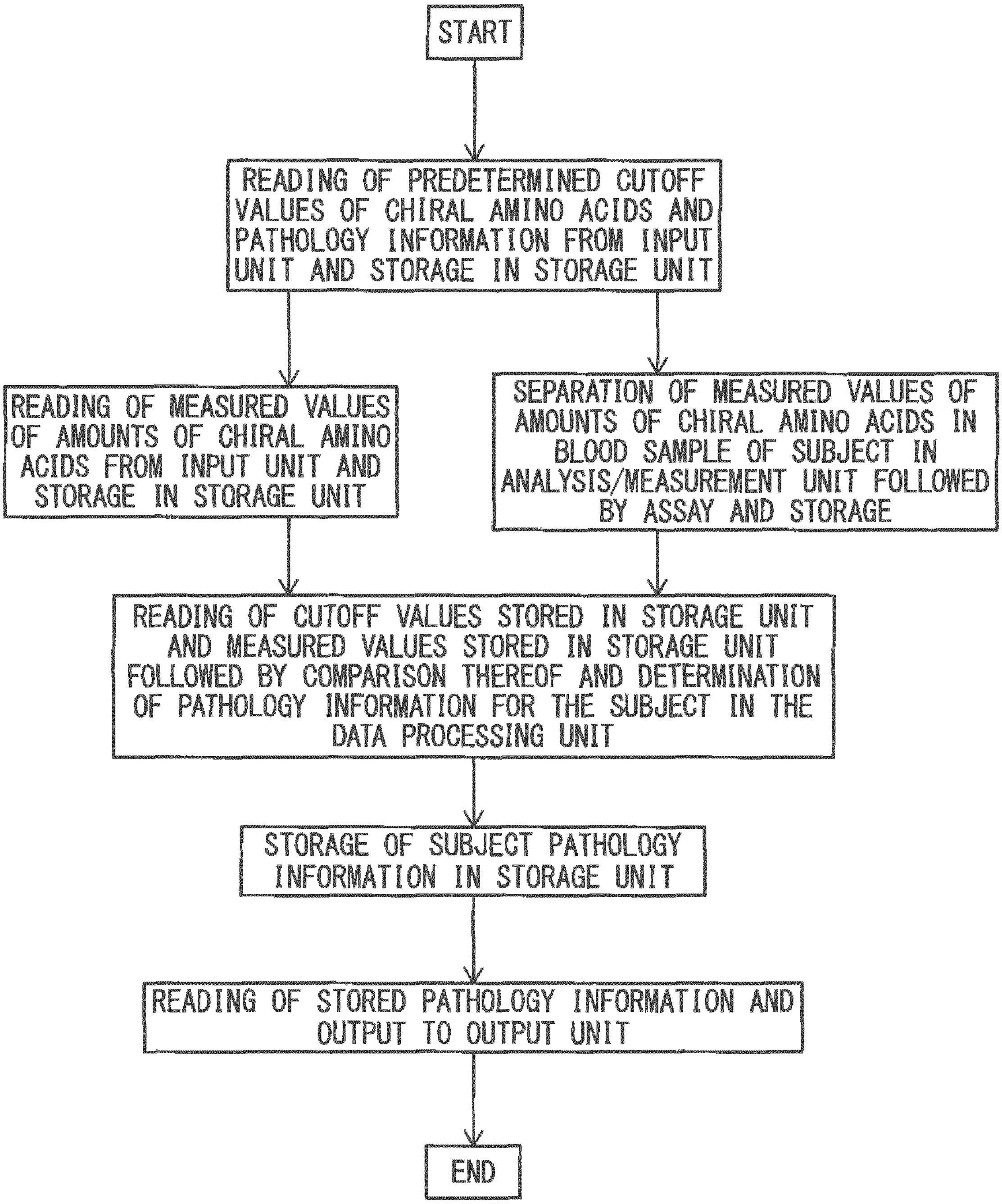
START
READING OF PREDETERMINED CUTOFF VALUES OF CHIRAL AMINO ACIDS AND PATHOLOGY INFORMATION FROM INPUT UNIT AND STORAGE IN STORAGE UNIT
READING OF MEASURED VALUES OF AMOUNTS OF CHIRAL AMINO ACIDS FROM INPUT UNIT AND STORAGE IN STORAGE UNIT
SEPARATION OF MEASURED VALUES OF AMOUNTS OF CHIRAL AMINO ACIDS IN BLOOD SAMPLE OF SUBJECT IN ANALYSIS/MEASUREMENT UNIT FOLLOWED BY ASSAY AND STORAGE
READING OF CUTOFF VALUES STORED IN STORAGE UNIT AND MEASURED VALUES STORED IN STORAGE UNIT FOLLOWED BY COMPARISON THEREOF AND DETERMINATION OF PATHOLOGY INFORMATION FOR THE SUBJECT IN THE DATA PROCESSING UNIT
STORAGE OF SUBJECT PATHOLOGY INFORMATION IN STORAGE UNIT
READING OF STORED PATHOLOGY INFORMATION AND OUTPUT TO OUTPUT UNIT
END

FIG. 7

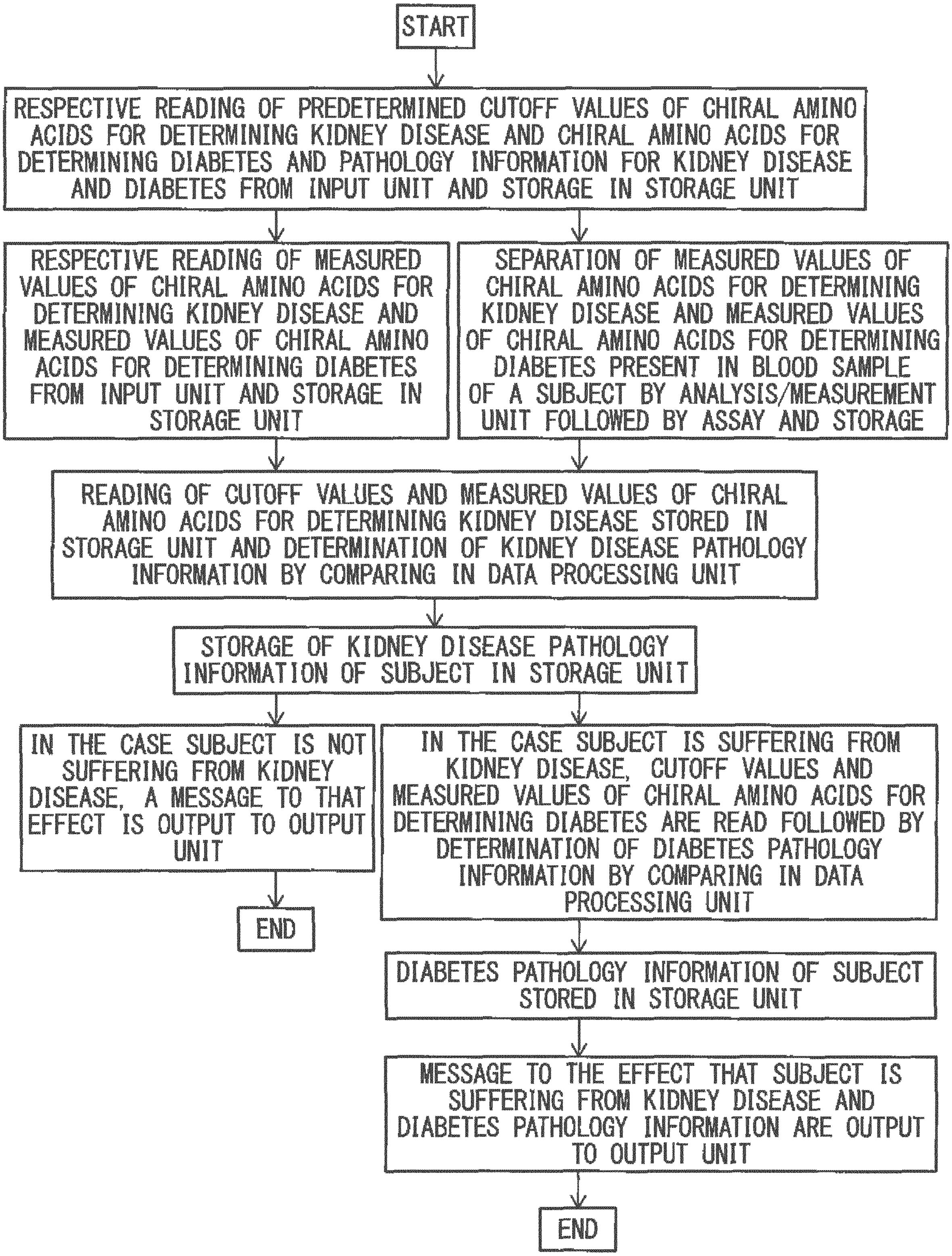
START
RESPECTIVE READING OF PREDETERMINED CUTOFF VALUES OF CHIRAL AMINO ACIDS FOR DETERMINING KIDNEY DISEASE AND CHIRAL AMINO ACIDS FOR DETERMINING DIABETES AND PATHOLOGY INFORMATION FOR KIDNEY DISEASE AND DIABETES FROM INPUT UNIT AND STORAGE IN STORAGE UNIT
RESPECTIVE READING OF MEASURED VALUES OF CHIRAL AMINO ACIDS FOR DETERMINING KIDNEY DISEASE AND MEASURED VALUES OF CHIRAL AMINO ACIDS FOR DETERMINING DIABETES FROM INPUT UNIT AND STORAGE IN STORAGE UNIT
SEPARATION OF MEASURED VALUES OF CHIRAL AMINO ACIDS FOR DETERMINING KIDNEY DISEASE AND MEASURED VALUES OF CHIRAL AMINO ACIDS FOR DETERMINING DIABETES PRESENT IN BLOOD SAMPLE OF A SUBJECT BY ANALYSIS/MEASUREMENT UNIT FOLLOWED BY ASSAY AND STORAGE
READING OF CUTOFF VALUES AND MEASURED VALUES OF CHIRAL AMINO ACIDS FOR DETERMINING KIDNEY DISEASE STORED IN STORAGE UNIT AND DETERMINATION OF KIDNEY DISEASE PATHOLOGY INFORMATION BY COMPARING IN DATA PROCESSING UNIT
STORAGE OF KIDNEY DISEASE PATHOLOGY INFORMATION OF SUBJECT IN STORAGE UNIT
IN THE CASE SUBJECT IS NOT SUFFERING FROM KIDNEY DISEASE, A MESSAGE TO THAT EFFECT IS OUTPUT TO OUTPUT UNIT
END
IN THE CASE SUBJECT IS SUFFERING FROM KIDNEY DISEASE, CUTOFF VALUES AND MEASURED VALUES OF CHIRAL AMINO ACIDS FOR DETERMINING DIABETES ARE READ FOLLOWED BY DETERMINATION OF DIABETES PATHOLOGY INFORMATION BY COMPARING IN DATA PROCESSING UNIT
DIABETES PATHOLOGY INFORMATION OF SUBJECT STORED IN STORAGE UNIT
MESSAGE TO THE EFFECT THAT SUBJECT IS SUFFERING FROM KIDNEY DISEASE AND DIABETES PATHOLOGY INFORMATION ARE OUTPUT TO OUTPUT UNIT
END

**INTERNATIONAL SEARCH REPORT**

International application No. PCT/JP2017/018592

A. CLASSIFICATION OF SUBJECT MATTER
*G01N33/68*(2006.01)i

According to International Patent Classification (IPC) or to both national classification and IPC

B. FIELDS SEARCHED

Minimum documentation searched (classification system followed by classification symbols)
G01N33/68

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/WPIDS/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | Hironori ISHIDA, "Serum D-Amino Acid Elucidated in Renal Failure", Kitasato Medicine, 1993, vol.23, pages 51 to 62 | 1,4<br>2-3,5-8 |
| X<br>Y | MEISS, E. et al., Metabolite targeting: development of a comprehensive targeted metabolomics platform for the assessment of diabetes and its complications, Metabolomics, 2016.02.09, Vol. 12, Issue 3, pp. 1-15 | 1,4<br>2-3,5-8 |
| X<br>Y | CHEN, T. et al., Changes of plasma free amino acids in type 2 diabetes, Diabetologia, 2009, Vol. 52, No. S1, pp. S322-S323 | 1<br>2-3,5-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August 2017 (07.08.17) | 15 August 2017 (15.08.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2017/018592

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2011/034088 A1 (Asahi Kasei Pharma Corp.), 24 March 2011 (24.03.2011), paragraphs [0034], [0051] to [0060], [0067] to [0077] (Family: none) | 2-3,5-8, 10-13 |
| X<br>Y | MENDEZ, J.A. et al., Age-related reference values for plasma amino acids in a Spanish population measureed by gas chromatography-mass spectrometry, Journal of pediatric endocrinology and metabolism, 2013.04.01, Vol. 26, Issue 3-4 | 9<br>10-13 |
| Y | JP 2011-092100 A (DNA Chip Research Inc.), 12 May 2011 (12.05.2011), claims; paragraph [0036] (Family: none) | 12-13 |
| A | JP 2013-224929 A (Kyushu University), 31 October 2013 (31.10.2013), claims; paragraphs [0066] to [0069], [0072] & US 2015/0079623 A1 claims; paragraphs [0240] to [0245], [0250] & WO 2013/140785 A1 & EP 2829877 A1 | 1-13 |

Form PCT/ISA/210 (continuation of second sheet) (January 2015)

## INTERNATIONAL SEARCH REPORT

International application No.
PCT/JP2017/018592

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

International application No. PCT/JP2017/018592

Continuation of Box No.III of continuation of first sheet(2)

The invention in claim 1 and the invention in claim 10 have a common technical feature of "a method for analyzing a blood sample which comprises measuring the amount of a chiral amino acid in the blood sample and comparing the thus measured value with a preset cutoff value to thereby determine the physical conditions".

However, document 1 (Hironori ISHIDA, "Serum D-Amino Acid Elucidated in Renal Failure", Kitasato Medicine, 1993, vol.23, pages 51 to 62 ) and document 5 (JP 2013-224929 A (Kyushu University et al.), 31 October 2013 (31.10.2013), claims; paragraphs [0067], [0072]) describe that the presence or absence of a disease in a subject can be judged by comparing a chiral amino acid concentration in the serum with a pathological index value. Thus, the aforesaid technical feature does not make a contribution over the prior art and, therefore, cannot be considered as a special technical feature.

Further, there is no other same or corresponding special technical feature between these inventions.

Moreover, document 5 (see, in particular, claims 2-3 and paragraph [0072]) describes that the presence or absence of diabetes in a subject can be judged by comparing a chiral amino acid concentration in the serum with a pathological index value.

Therefore, to diagnose diabetes on the basis of the measured amount of a chiral amino acid in a blood sample cannot be considered as a special technical feature of invention.

Consequently, the inventions as indicated below are involved in claims 1-13 of this application.

(Invention 1) Inventions in the inventions in claims 1-8 wherein the special technical feature of invention is to use "D-aspartic acid" in a blood sample as "an indicator for diabetes".

(Invention 2) Inventions in the inventions in claims 1-8 wherein the special technical feature of invention is to use "D-proline" in a blood sample as "an indicator for diabetes".

(Invention 3) Inventions in the inventions in claims 1-8 wherein the special technical feature of invention is to use "L-glutamine" in a blood sample as "an indicator for diabetes".

(Invention 4) Inventions in the inventions in claims 1-8 wherein the special technical feature of invention is to use "L-isoleucine" in a blood sample as "an indicator for diabetes".

(Invention 5) Inventions in claims 9-13 relating to a method for analyzing a blood sample for age estimation and a blood analysis system for the method.

Claims 9-13 are not relevant to inventions which involve all of the matters to define the invention in claim 1 and which have a same category.

Further, as a result of the search which has been carried out with respect to claims classified into Invention 1, claims 9-13 are not relevant to inventions on which it is substantially possible to carry out a search without an additional prior-art search and judgment, and there is no other reason for that it can be considered that it is efficient to carry out a search on said claims 9-13 together with claims 1-8, and consequently, it is impossible to classify claims 9-13 into Invention 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5876826 B
- JP 5571696 B
- JP 5698254 B
- JP 4291628 B
- WO 2013140785 A
- JP 2009184981 A


### Non-patent literature cited in the description


- *Transl. Res.,* April 2012, vol. 159 (4), 303-12
- **HAMASE, K. ; ZAITSU, K.** *Analytical Chemistry,* 2004, vol. 53, 677-690
- *Journal of Chromatography. B, Analytical technologies in the biomedical and life science,* 2014, vol. 966, 187-192
- *J. Chromatogr. A,* 2010, vol. 1217, 1056-1062
- *Journal of Chromatography, B: Analytical technologies in the biomedical and life sciences,* 2009, vol. 877, 2506-2512